# EUROPEAN PATENT SPECIFICATION

(11) **EP 3 927 177 B1**
(45) Date of publication and mention of the grant of the patent: **14.02.2024**
(21) Application number: 20705977.5
(22) Date of filing: 20.02.2020
(51) Int. Cl.: A23K 10/30, A23K 20/111, A23K 20/163, A23K 50/10, A23K 50/30, A61K 31/353, A61K 31/7048, A61P 37/02, A61K 31/352

(54) **FLAVONOIDS AND ANIMAL HEALTH AND PERFORMANCE**
FLAVONOIDE UND TIERGESUNDHEIT UND -LEISTUNG
FLAVONOÏDES ET SANTÉ ET PERFORMANCE DES ANIMAUX

(30) Priority: 21.02.2019 EP 19382127
(43) Date of publication of application: 29.12.2021
(73) Proprietor: HealthTech Bio Actives, S.L.U., 08029 Barcelona (ES)
(72) Inventor: CRESPO MONTERO, Francisco Javier, 08173 SANT CUGAT DEL VALLÈS (ES); PANIAGUA JIMÉNEZ, Montserrat, 08006 BARCELONA (ES); ARÍS GIRALT, Anna, 08140 CALDES DE MONBUI (ES); DEVANT GUILLE, Maria, 08140 CALDES DE MONTBUI (ES)
(74) Representative: ZBM Patents - Zea, Barlocci & Markvardsen
(86) International application number: PCT/EP2020/054479
(87) International publication number: WO 2020/169733

(56) References cited:
- US-A1- 2010 136 161
- US-A1- 2010 291 239
- US-A1- 2015 362 481
- WIBKE S. U. ROLAND ET AL: "Bitter Taste Receptor Activation by Flavonoids and Isoflavonoids: Modeled Structural Requirements for Activation of hTAS2R14 and hTAS2R39", JOURNAL OF AGRICULTURAL AND FOOD CHEMISTRY, vol. 61, no. 44, 6 November 2013 (2013-11-06), pages 10454-10466, XP055297209, US ISSN: 0021-8561, DOI: 10.1021/jf403387p
- MEYERHOF WOLFGANG ET AL: "The molecular receptive ranges of human TAS2R bitter taste receptors", CHEMICAL SENSES, IRL PRESS, OXFORD, GB, vol. 35, no. 2, 1 February 2010 (2010-02-01), pages 157-170, XP009139503, ISSN: 0379-864X, DOI: 10.1093/CHEMSE/BJP092 [retrieved on 2009-12-18]
- TEPPER BEVERLY J ET AL: "Genetic variation in taste sensitivity to 6-n-propylthiouracil and its relationship to taste perception and food selection", ANNALS OF THE NEW YORK ACADEMY OF SCIENCES, NEW YORK ACADEMY OF SCIENCES, US, vol. 1170, 1 July 2009 (2009-07-01), pages 126-139, XP002576914, ISSN: 0077-8923, DOI: 10.1111/J.1749-6632.2009.03916.X

## Description

This application claims the benefit of European Patent Application 19382127.9 filed on February 21st, 2019.

### TECHNICAL FIELD

The present invention relates to the use of naringin, its aglycone form, naringenin, or mixtures thereof in the modulation of the expression of some specific gene receptors in ruminants and/or pigs which positively impact on feeding behaviour, intake patterns and animal behaviour.

### BACKGROUND ART

Phytochemicals are chemical substances found in vegetables and edible fruits. They play important functions in plants, acting as protecting molecules from harmful agents (insects, bacteria...) or stressful situations (UV, temperature, lack of water...). Otherwise, phytochemicals have showed biological activities and healthy effects in humans and animals.

Flavonoids are polyphenols that have been deeply studied, and *Citrus* fruits are considered the major source of flavonoids, containing a wide range of these phytochemicals. Some of these compounds have anti-inflammatory, antioxidant, and antimicrobial properties. Due to their interesting capabilities, flavonoids from different sources are being studied for different applications in animal production. Bioflavex^{®} CA (Interquim, S.A., Spain) is an extract from bitter orange (*Citrus aurantium*) whose major flavonoid is naringin. Naringin is a glycosylated flavanone classified into the neohesperidoside type, with a neohesperidose (rhamnosyl-α-1,2 glucose) attached to its basic structure as a flavanone. Other extracts containing naringin have been shown to have beneficial effects in regulating rumen pH in fattening beef, as well as reducing in vitro methane production from steers fed high concentrate diets. Properties of naringin may affect rumen microflora, increasing the concentration of bacteria which consume lactic acid such as *Megasphaera elsdenii* resulting in a higher ruminal pH, and a depression of methanogenic archaea communities (PCT application number WO2013156574). Rumen volatile fatty acids (VFA) composition has been modified as well, increasing molar proportion of propionic acid. As propionic acid is an important regulator of feed intake in ruminants fed high-starch diets, affecting both satiety and hunger, the supplementation of flavonoids could affect eating pattern of bulls fed high-concentrate diets. Moreover, this supplementation could reduce methane production, and together with the reduced ruminal pH fluctuations could increase efficiency of nutrient utilization in steers.

However, the mechanisms whereby citrus flavonoids may modulate eating and animal behaviour are unknown. Naringin is the flavonoid responsible of the typical bitter taste in some citrus fruits, so another possible mechanism may be related to bitter taste, one of the five basic tastes (sweet, salty, bitter, soar and umami) perceived by humans and animals. Recent studies have demonstrated that taste receptors, including bitter taste receptors (TAS2R), are expressed in gastrointestinal tract, including ruminants and swine. Thus, bitter receptors may modulate the eating pattern of animals, reducing large meal sizes, improving digestive epithelium health parameters and enhancing animal welfare as a result. Nutrition gastrointestinal tract can have an impact on animal behavior modulating sexual and aggressive behaviors as well as oral non-nutritive behaviors, since there is a link between the digestive tract and the central nervous system, also known as gut-brain axis where digestive microbiota is a key player.

Several solutions have been proposed so far in the state of the art in order to improve behaviour and, consequently, welfare in animals. However, it is still difficult to obtain natural substances that possess long term effects in the animals, since the animals seem to get used to them, and as consequence disappears their positive effects over time.

US2010/291239A1 discloses the use of naringin for improving ruminal fermentation.

US2010/136161 an animal feed that contain at least naringine dihydrochalcone for improving appetite or performance in live stock.

Finally, Wibke S.U. Roland et al., in Journal of Agricultural and Food Chemistry, 2013, vol. 61, pp10454-10466 discloses the activation of bitter receptor hTAS2R39 in human by naringenin. Meyerhof Wolfgang et al., in Chemical Senses, IRL Press, Oxford, GB, 2010, vol. 35, no. 2, pp 157-170 discloses that naringin does not trigger any response on some hTAS2Rs in human. Tepper Beverly J et al., in Annals of the New Your Academy of Sciences, 2009, vol. 1170. pp. 126-139 discusses the taste perception of grapefruit juice with naringin. US2015/362481 discloses the use of naringin as bitter tasting compound (or bitter agonist), as it activates some human bitter receptors.

Therefore, there is a need in the art for new methods, which are able to provide targeted modulation of the gene expression of some specific gene receptors involved in gut-brain axis, namely, bitter taste receptors and immunity receptors, which positively impact on feeding behaviour, gut microflora, inflammatory response and animal behaviour.

Furthermore, it is an object of the invention to provide alternative animal feed compositions comprising compounds of natural origin and which are safe for its use in livestock farming and effective in improving eating behaviour and its impact on digestive disturbences, the animal behaviour and, therefore, the welfare of animals.

The above issues have been addressed in the present invention and specific embodiments thereof.

### BRIEF DESCRIPTION OF DRAWINGS

Figure 1 represents non-agonistic interactions of bulls fed high-concentrate diets with or without Bioflavex^{®} CA supplementation.
Figure 2 represents agonistic interactions of bulls fed high-concentrate diets with or without Bioflavex^{®} CA supplementation.
Figure 3 represents sexual interactions of bulls fed high-concentrate diets with or without Bioflavex^{®} CA supplementation.
Figure 4 represents the relative gene expression ratio in rumen of Holstein bulls fed high-concentrate diets supplemented or not with Bioflavex CA after 168 days of treatment.
Figure 5 represents the relative gene expression ratio of TAS2R in the jejunum of piglets after 7 days fed with two diets which correspond T1: basal diet and T2: T1 + bitter orange extract (300g/Tm).
Figure 6 represents the relative gene expression ratio in duodenum of Holstein bulls fed high-concentrate diets supplemented or not with Bioflavex CA after 168 days of treatment.

### SUMMARY OF THE INVENTION

In a first aspect, the present invention relates to a non-therapeutic use of naringin, naringenin or mixtures thereof for the targeted modulation of the gene expression of at least one bitter taste receptor and/or at least one gut-brain axis receptor to modify the eating pattern and rumination in ruminants or for the targeted modulation of the gene expression of at least one bitter taste receptor to modify the eating pattern in pigs, wherein the non-therapeutic use comprises administering naringin, naringenin or mixtures thereof, wherein the amount of naringin, naringenin or mixtures thereof is administered as a mixture with feed, wherein said feed composition comprises at least 0.005% w/w of naringin or the equimolar amount of naringenin or of a mixture of naringin and naringenin if both are present.

In a second aspect, the present invention relates to naringin, naringenin or mixtures thereof for use in the modulation of the gene expression of at least one immune-related gene in ruminants and/or pigs, wherein said product is administered as a mixture with feed, wherein said feed composition comprises at least 0.005% w/w of naringin or the equimolar amount of naringenin or of a mixture of naringin and naringenin if both are present.

### DETAILED DESCRIPTION OF THE INVENTION

The present invention relates to the use of naringin, its aglycone form, naringenin, or mixtures thereof in the modulation of the expression of some specific gene receptors in ruminants and/or pigs which positively impact on feeding behaviour, intake patterns and animal behaviour.

In a first aspect, the present invention relates to a non-therapeutic use of naringin, naringenin or mixtures thereof as defined above for the targeted modulation of the gene expression of at least one bitter taste receptor and/or at least one gut-brain axis receptor, in ruminants and/or pigs, comprising administering naringin, naringenin or mixtures thereof.

The term "targeted modulation of gene expression" as used herein is meant to include a reduction of the gene expression as well as an amplification of said gene expression.

The authors of the present invention have now found that naringin, naringenin or mixtures thereof administered to ruminants and/or pigs, significantly modify the expression of some genes in the rumen epithelium in the case of ruminants or intestine in the case of pigs involved in taste perception, inflammation and gut-brain crosstalk mechanisms. This effect explains the differences observed in eating pattern and animal behaviour in ruminants and/or pigs.

Furthermore, it was also surprisingly discovered that ruminants supplemented with naringin, naringenin or mixtures thereof modified their eating pattern reducing large meal sizes and spending more time eating straw and the rumen wall health parameters were improved. In addition, it was also observed that ruminants supplemented with naringin, naringenin or mixtures thereof reduced agonistic behaviour and sexual interactions.

### Bitter taste receptor

In one embodiment of the present invention, the bitter taste receptor is selected from the group consisting of TAS2R7, TAS2R16, TAS2R38 and TAS2R39.

Taste receptors were initially discovered in taste buds located in the tongue and different parts of the oral cavity. Recently, many studies describe the presence of taste receptors for the basic tastes (sweet, bitter, umami, soar and salty) throughout the body, including respiratory system and gastrointestinal tract. This peripheral gustatory system would have the function of tasting the luminal content of the digestive tract, and regulating nutrient transporters expression, nutrients uptake and also the release of gut hormones and neurotransmitters involved in the regulation of the gastrointestinal function, feeding and satiety.

Bitter molecules trigger the release of anorexigenic hormones and peptides, as cholecystokinin (CCK), neuropeptide Y (NPY), and peptide YY (PYY). This would be a logical response, as bitter taste has been often related to the presence of toxins, and is considered to have a negative value. Thus, the activation of an anorexygenic response in the digestive tract would be an adaptive response to this taste. Eating and animal (social and sexual) behaviour could be related. It has been described that some nutritional strategies focused on increasing time devoted to eat reduced aggressive and abnormal behaviour in animals.

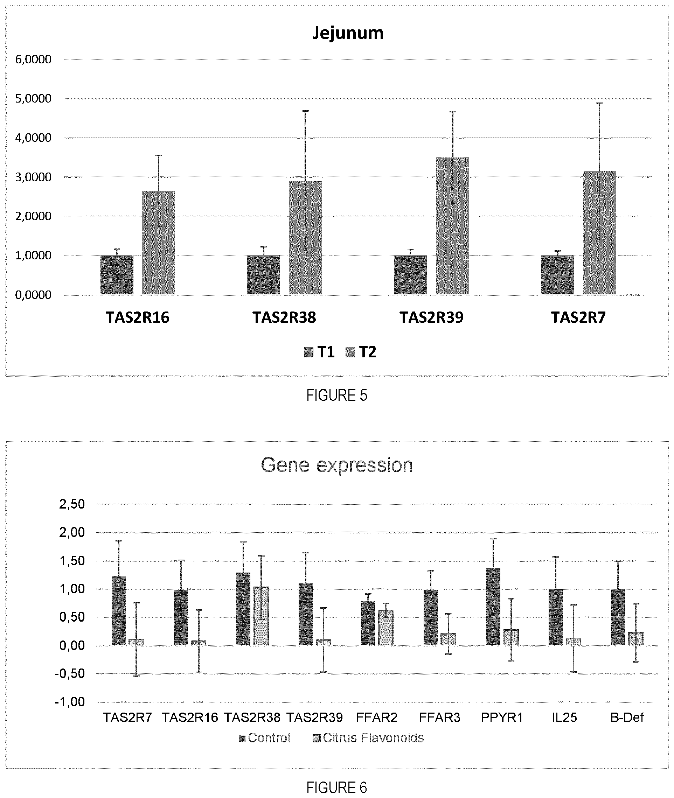

The authors of the present invention found that bulls supplemented with naringin, naringenin or mixtures thereof dedicated more time to eat concentrate or ruminating during the visual scan procedure. See, for example, animal behaviour disclosed in Example 1 (Tables 7 and 8). Thus, animals devoting more time to feeding events had less time to perform other behaviour, as aggressive and sexual interactions. Herein, bitter taste receptors in the digestive tract play an important role modulating eating behaviour and in consequence animal behaviour. Furthermore, surprisingly, flavonoids supplementation modified gene expression of all bitter taste receptors analyzed in the rumen wall (*TAS2R7, TAS2R16, TAS2R38,* and *TAS2R39*)*,* as it can be seen in Example 1 (Table 11) as well as in the duodenum epithelium, as it can be seen in Example 3 (Figure 6). Although naringin has a characteristic bitter taste, it is rapidly deglycosylated by enzymes to naringenin, and rumen microflora is able to degrade naringin to naringenin as well. Contrary to naringin, naringenin acts as an important bitter masking molecule, and this property is well known in food industry, mainly for its use in beverages (US 8,685,436). It has been described how some flavanones act as actual antagonists for human bitter taste receptor TAS2R39, reducing the receptor response possibly by orthosteric mechanism acting over a single binding pocket of the bitter taste receptor. Example 1 and Example 3 showed a reduction in the gene expression of the four bitter taste receptors analyzed in bulls supplemented with citrus flavonoids (*TAS2R7, TAS2R16, TAS2R38,* and *TAS2R39*)*.* These results would agree with the function of naringenin as a bitter masking molecule, acting as antagonist for different bitter taste receptors at the same time. This reduction in the gene expression of bitter taste receptors analyzed could be related with the greater time devoted to eat observed in bulls feeded with Bioflavex^{®}. Thus, supplementation with citrus flavonoids in bulls modulates eating pattern acting regulating bitter taste receptors expressed in the rumen wall and duodenum, and consequently modifying the release of hormones and bioactive peptides involved in hanger and satiety. Therefore, citrus flavonoids might act as bitter masking molecules in the rumen, and bulls supplemented with these flavonoids devoted more time to eat, performing lesser agonist and sexual interactions.

In the case of swine, when citrus flavonoids were added to the feed of weaned piglets the gene expression of all the bitter taste receptors analyzed (TAS2R7, TAS2R16, TAS2R38, and TAS2R39) were greater at the jejunum compared with non-supplemented animals as it can be seen in Example 2, Figure 5.

The term "flavonoids" as used herein refers to a class of water-soluble vegetable pigments producing yellow or red/blue pigmentation in petals. The term "flavanones" refers to a type of flavonoids. Flavanones are generally glycosylated by a disaccharide at position seven to give "flavanone glycosides".

### Gut-brain axis receptor

In another embodiment of the present invention, the gut-brain axis receptor is selected from the group consisting of FFAR2, FFAR3, PPYR1 and CCKBR.

Gut-brain axis has been proposed as a communication network between digestive system and brain, and may affect behaviour in humans and other animals. It has been suggested that the rumen could be involved in the crosstalk between digestive system and brain in beef cattle, and modifying animal aggressive and sexual behaviour could be implicated in this behaviour modulation. They observed that diet modified the expression of different genes (*FFAR3, PPYR1, ADRA2C, occluding* and *TNFα*) in rumen of cattle fed high-concentrate diets that may be involved in the gut-brain axis. These authors suggested that the rumen could have an important role in the crosstalk between digestive system and brain, and modifying animal aggressive and sexual behaviour. Surprinsingly, the results of the present invention have showed a clear decline in the gene expression of receptors related with neurotransmitters, as *CCKBR* (acts as *CCK* and *gastrin* receptor) and *PPYR* (acts as *NPY* and *PYY* receptor) in bulls supplemented with flavonoids, as it can be seen in Example 1 (Table 11) and Example 3 (Figure 6). These results would be in concordance with the reduction in the expression of the bitter taste receptors analyzed in the rumen wall and duodenum of these animals, because of these receptors are involved in the release of these anorexigenic molecules.

In another embodiment of the present invention, the gene expression of at least one said receptor is reduced. In a preferred embodiment of the present invention, the gene expression of at least one said receptor is reduced at least 20%, preferably is reduced at least 30%, more preferably is reduced at least 40%.

As it is shown in the examples below, the inventors have surprisingly found that expression of bitter taste receptors and gut-brain axis receptors are significantly reduced by administering to ruminants a feed composition according to the present invention in respect to those not fed with such a composition.

The term "ruminant" as used herein refers to any artiodactyl mammal of the suborder *Ruminantia.* Said mammals chew the cud and have a stomach of four compartments, one of which is the rumen. The group includes, among others, cattle, buffalo, sheep, deer, camels, goats and antelopes. The term 'cattle' should be understood to include cows, calves and bulls. In another embodiment of the present invention, the ruminants of the first aspect are cattle, buffalo, sheep, deer, camels, goats or antelopes. In a preferred embodiment of the present invention, said ruminants are cattle.

The term "pig" as used herein refers to any mammal of the suidae family. Said suids are stout animals with small eyes and coarse, sometimes sparse, hair. All have muzzles ending in a rounded cartilage disk used to dig for food. Some species have tusks. Suids are omnivorous and usually gregarious. The group includes, among others, pigs, wild or domestic swines, hogs and boars. In another embodiment of the present invention, the pigs of the first aspect are wild or domestic swine, hogs or boars.

The amount of naringin, naringenin or mixtures thereof is administered as a mixture with feed, wherein said feed composition comprises at least 0.005% w/w of naringin or the equimolar amount of naringenin or of a mixture of naringin and naringenin if both are present.

In a preferred embodiment of the present invention, said feed composition comprises at least 0.01% w/w of naringin or the equimolar amount of naringenin or of a mixture of naringin and naringenin if both are present when it is administered to ruminants.

In another preferred embodiment of the present invention, said feed composition comprises at least 0.0075 % w/w of naringin or the equimolar amount of naringenin or of a mixture of naringin and naringenin if both are present when it is administered to pigs.

Naringin is a flavanone-7-O-glycoside between the flavanone naringenin and the disaccharide neohesperidose. The flavonoid naringin occurs naturally in citrus fruits, especially in grapefruit, where naringin is responsible for the fruit's bitter taste. In commercial grapefruit juice production, the enzyme naringinase can be used to remove the bitterness created by naringin. In humans naringin is metabolized to the aglycone naringenin (not bitter) by naringinase present in the gut. Naringenase is a multienzyme complex which possesses α-L--rhamnosidase and β-d-glucosidase active centers. This happens in two steps, first, naringin is hydrolyzed by α-L-rhamnosidase activity of naringinase to rhamnose and prunin. The prunin formed is then hydrolyzed by β-d-glucosidase activity of naringinase into naringenin and glucose (see Scheme 1). Naringenase is an enzyme that has widely ocurrence in nature and it is produced by many microorganisms mainly fungi, yeast and bacteria. While glucosides can be cleaved by intestinal lactase-phlorizin hydrolase or small intestinal epithelial cell β-glucosidase, no human α-L-rhamnosidase or rutinosidase exists and the bioavailability of rhamnose containing flavonoids is fully dependent on their cleavage by intestinal microbiota. In pigs these enzymes can also be found in gut. However in ruminants they are in the rumen.

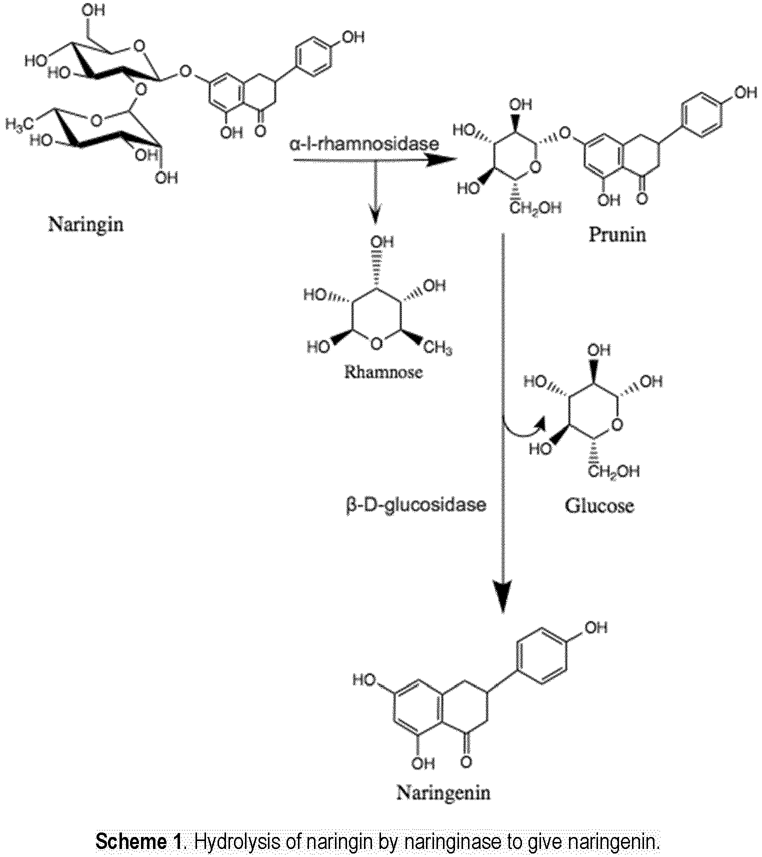

Naringenin has the skeleton structure of a flavanone with three hydroxy groups at the 4', 5, and 7 carbons. It may be found both in the aglycol form, naringenin, or in its glycosidic form, naringin, which has the addition of the disaccharide neohesperidose attached via a glycosidic linkage at carbon 7.

This equimolar amount of naringenin or of a mixture of naringin and naringenin is meant, for example, the amount of naringenin equivalent in % w/w to naringin considering the molecular weight. For example, 0.01 % w/w of naringin will be equivalent to 0.0053 % w/w of naringenin.

In another preferred embodiment of the present invention, said feed composition is administered in the form of mash or pellet.

The manner of feeding is not restricted to any in particular, and the feed composition of the present invention may be given by top-dressing over the compound feed, or fed after the present feed composition is mixed with the compound feed.

The shape of the feed composition used in the present invention is not restricted to any in particular and may be in any form of a conventional feed composition, such as a powder or mash and a pellet. Also, said feed composition may be produced according to the generally employed method for producing a compound feed and a feed supplement

The composition used in the present invention can contain other feed ingredients such as vitamins, enzymes, mineral salts, ground cereals, protein-containing components, carbohydrate-containing components, wheat middlings and/or brans.

The feed composition used in the present invention can be any kind of animal feed, whose possible compositions are well known to the skilled in the art, designed according to the nutrition requirements of the specific animals and the specific age period. For example, a feed for piglets typically contains cereals, such as corn, wheat, soybeans, barley or oats; different protein sources, such as fishmeal, soybean meal or animal plasma, for example; amino acids, such as methionine, threonine, valine, tryptophan, arginine, histidine or leucine; as well as vitamins and minerals to meet the requirements for growth of piglets (U.S. National Research Council, NRC, 2012).

In another embodiment of the present invention, naringin, naringenin or mixtures thereof are administered in a daily dose of 0.001 to 0.01 g of naringin per kg body weight of the animal or the equimolar amount of naringenin or of a mixture of naringin and naringenin if both are present. In a preferred embodiment of the present invention, naringin, naringenin or mixtures thereof are administered in a daily dose of 0.002 to 0.003 g of naringin per kg body weight of the animal or the equimolar amount of naringenin or of a mixture of naringin and naringenin if both are present.

In another embodiment of the present invention, naringin, naringenin or mixtures thereof is in the form of a natural plant extract.

In another preferred embodiment of the present invention, said natural plant extract is bitter orange extract. In another preferred embodiment of the present invention, said bitter orange extract additionally comprises at least one flavanone glycoside selected from the group consisting of neohesperidin, isonaringin, poncirin, hesperidin and mixtures thereof.

In another preferred embodiment of the present invention, said bitter orange extract is a mixture comprising 20 to 35 % w/w of naringin, 10% to 20% w/w of neohesperidin and 0,5% to 5% w/w of poncirin, preferably, said bitter orange extract comprises 21 to 30 % w/w of naringin, 11 to 18 % w/w. of neohesperidin and 1 to 5 % w/w of poncirin, more preferably said bitter orange extract comprises 25 to 27 % w/w of naringin, 11 to 15 % w/w of neohesperidin and 1 to 3 % w/w of poncirin. In a particular case, said natural plant extract is commercially available (Bioflavex^{®}).

Therefore, according to the present invention, the naringin, naringenin or mixtures thereof of the present invention can be obtained from a plant, more particularly, from a citrus plant. All the propducts according to the present invention are products of natural origin and easily obtainable.

The term "citrus" as used herein refers to a plant of the genus Citrus. Examples of said citrus plants include *Citrus maxima* (Pomelo), *Citrus medica* (Citron) *Citrus reticulata* (Mandarin orange), *Citrus aurantium* (Bitter orange), *Citrus latifolia* (Persian lime), *Citrus limon* (Lemon) *Citrus paradisi* (Grapefruit), *Citrus sinensis* (Sweet orange), *Citrus trifoliata* (Trifoliate Orange), etc.

Methods for the isolation of flavanoids from plants are well known in the state of the art. In a particular case, the bitter orange extract can be obtained from ground citrus fruits (especially *Citrus aurantium*) by ordinary methods well known by the skilled person in the art such extraction, filtration, concentration, precipitation, clarification and final drying. Extraction processes can be performed in binary alkanol/water systems, wherein the alkanol is selected from methanol, ethanol, propanol and the like. Methanol is preferably used. As an illustrative, non-limitative, example, 50 g of dried bitter orange are extracted with 300 ml of methanol. The suspension is centrifuged to separate the residue and the mother liquor is vacuum concentrated to a final volume of 50 ml. The resulting liquid is allowed to stand at room temperature for five days, filtered-off to separate insoluble material, concentrated, filtered again through a diatomaceous earth bed and spray-dried.

In a particular embodiment, said flavanone can be obtained from the fruit of a citrus plant. For example, naringin is a glycosylated flavanone obtained from the peel of some citric fruits such as grapefruit (*Citrus paradise*) and bitter orange (*Citrus aurantium*)*.* It is also found in the pulp of the fruit and in the leaves, flowers and seeds of the plant. Illustrative, non-limitative, methods for the isolation of the flavonoids according to the present invention are, for example, those described in documents US2421063A and US2421062A wherein a method for the recovery of naringin from plant material is described. Also, hesperidin can be obtained according to the methods described in documents US2442110A, US2348215A and US2400693A. Likewise, neohesperidin can be obtained according to the method described in document US3375242A. US3375242A describes a method for producing neohesperidin wherein naringin is reacted with isovanillin to produce neohesperidin chalcone. This chalcone is then cyclised to yield neohesperidin.

Additionally, the flavonones of the composition of the present invention can be easily obtained since they are commercially available. For example, as it is shown in the examples accompanying the present invention, isonaringin, neoeritrocin and poncirin are purchased from INDOFINE Chemical Company, Inc (USA). Also, as described above, said natural plant extract according to the present invention is commercially available (Bioflavex^{®}).

In another embodiment of the present invention, said bitter orange extract is administered in a daily dose of 0.005 g per kg body weight of the animal to 0.02 g per kg body weight of the animal, preferably said bitter orange extract is administered in a daily dose of 0.01 g per kg body weight of the animal.

In a further embodiment of the present invention, comprises administering naringin, naringenin or mixtures thereof to a ruminant and/or pigs for at least 5 days. In a preferred embodiment of the present invention, comprises administering naringin, naringenin or mixtures thereof to pigs for at least 10 days. In another preferred embodiment, comprises administering naringin, naringenin or mixtures thereof to ruminants for at least 15 days.

### Immune-related genes

In a second aspect, the present invention relates to naringin, naringenin or mixtures thereof for use in the modulation of the gene expression of at least one immune-related gene in ruminants and/or pigs.

The term "modulation of gene expression" as used herein is meant to include a reduction of the gene expression as well as an amplification of said gene expression. It could also be understood that such modulation of the gene expression could result also in a modulation of the production of at least one immune-related genes in ruminant and/or pigs. Thus, a reduction of the gene expression of any immune-related gene will result in a reduction of the production of said gene and further a modulation of inflammation.

Moreover, the authors of the present invention found that citrus flavonoids supplementation clearly reduced the gene expression of the receptors related with the inflammation in the rumen wall of the bulls. Inflammation has been suggested to play an important role in animal welfare and behaviour, possibly by the gut-brain axis crosstalk. Inflammation could be involved in a decrease of serotonin levels in serum, because of the increase in the use of the amino acid tryptophan to produce kynurine. Serotonin is a neurotransmitter that plays an important role within the gut-brain axis, and has been associated with mood modulation and a reduction in aggressive behaviors. Additionally, selective serotonin reuptake inhibitors (which increase extracellular serotonin) have been related to libido reduction and sexual problems in humans. *IL-25* is produced by a variety of cells, including immune and non-immune cells (epithelial and endothelial) and it can potentiate allergic inflammation. *Defensin-β* is an antimicrobial peptide, and have modulatory effects on innate and adaptive immune processes in mammals. Thus, Example 1 (see Table 11) and Example 3 show that citrus flavonoids supplementation reduced the gene expression of these pro-inflammatory molecules, and this could be leading to a reduction in inflammatory response and inflammation in the rumen wall and duodenum. Additionally, naringenin acts as a potent antioxidant and its anti-inflammatory effect is in accordance to the prior art. Moreover, the results obtained, as listed in Table 7 and 8, show aggressive and sexual interactions in bulls supplemented with citrus flavonoids were reduced, and rumen gene expression data would support that the reduction of the rumen inflammation could be a key player in this response.

In summary, concentrate intake regulation (time devote to eat) together with the inflammation and other gut-brain crosstalk mechanisms in the rumen wall are involved in the improvement of animal behaviour and welfare of animals, supplemented with citrus flavonoids.

The term "animal welfare" as used herein includes three elements: The animal's normal biological functioning (which, among other things, means ensuring that the animal is healthy and well-nourished), its emotional state (including the absence of negative emotions, such as pain and chronic fear), and its ability to express certain normal behaviour. This notwithstanding, not all behaviour are equally important in terms of animal welfare. From a practical standpoint, the clearest indication that a given behaviour is important is whether the animal shows a stress response or exhibits abnormal behaviour when prevented from performing it. A sow's prepartum nesting behaviour or the foraging behaviour of pigs are examples of such important behaviour. These three principles do not necessarily contradict one another; indeed, they are often complementary.

In one embodiment of the second aspect, the immune-related gene is selected from the group consisting of IL-6, IL-8, IL-10, IL-25, and β-defensin

In another embodiment of the second aspect, the gene expression of at least one said receptor is reduced. In a preferred embodiment of the present invention, the gene expression of at least one said receptor is reduced at least 20%, preferably is reduced at least 30%, more preferably is reduced at least 40%.

As it is shown in the examples below, the inventors have surprisingly found that expression of immune-related gene are significantly reduced by administering to ruminants a feed composition according to the present invention in respect to those not fed with such a composition.

In another embodiment of the second aspect, said ruminants are cattle, buffalo, sheep, deer, camels, goats or antelopes. In a preferred embodiment of the present invention, said ruminants are cattle.

In another embodiment of the second aspect, said pigs are wild or domestic swine, hogs or boars.

The product is administered as a mixture with feed, wherein said feed composition comprises at least 0.005% w/w of naringin or the equimolar amount of naringenin or of a mixture of naringin and naringenin if both are present. The product is meant to include naringin, naringenin or mixtures thereof.

In a preferred embodiment of the second aspect, said feed composition comprises at least 0.01 % w/w of naringin or the equimolar amount of naringenin or of a mixture of naringin and naringenin if both are present when it is administered to ruminants.

In another preferred embodiment of the second aspect, said feed composition comprises at least 0.0075 % w/w of naringin or the equimolar amount of naringenin or of a mixture of naringin and naringenin if both are present when it is administered to pigs.

In another preferred embodiment of the second aspect, said feed composition is administered in the form of mash or pellet.

In another embodiment of the second aspect, the product is administered in a daily dose of 0.001 to 0.01 g of naringin per kg body weight of the animal or the equimolar amount of naringenin or of a mixture of naringin and naringenin if both are present. In a preferred embodiment of the second aspect, the product is administered in a daily dose of 0.002 to 0.003 g of naringin per kg body weight of the animal or the equimolar amount of naringenin or of a mixture of naringin and naringenin if both are present.

In another embodiment of the second aspect, said product is in the form of a natural plant extract. In another preferred embodiment of the second aspect, said natural plant extract is bitter orange extract. In another preferred embodiment, said bitter orange extract additionally comprises at least one flavanone glycoside selected from the group consisting of neohesperidin, isonaringin, poncirin, hesperidin and mixtures thereof.

In another preferred embodiment of the second aspect, said bitter orange extract is a mixture comprising 20 to 35 % w/w of naringin, 10% to 20% w/w of neohesperidin and 0,5% to 5% w/w of poncirin, preferably, said bitter orange extract comprises 21 to 30 % w/w of naringin, 11 to 18 % w/w. of neohesperidin and 1 to 5 % w/w of poncirin, more preferably said bitter orange extract comprises 25 to 27 % w/w of naringin, 11 to 15 % w/w of neohesperidin and 1 to 3 % w/w of poncirin.

In another embodiment of the second aspect, said bitter orange extract is administered in a daily dose of 0.005 g per kg body weight of the animal to 0.02 g per kg body weight of the animal, preferably said bitter orange extract is administered in a daily dose of 0.01 g per kg body weight of the animal.

In a further embodiment of the second aspect, comprises administering said product to a ruminant and/or pigs for at least 5 days. In a preferred embodiment of the second aspect, comprises administering said product to pigs for at least 10 days. In another preferred embodiment, comprises administering said product to ruminants for at least 15 days.

In a further embodiment of the second aspect relates to naringin, naringenin or mixtures thereof for use in the treatment or prevention of diseases which have an inflammatory response in ruminants and/or pigs.

The main inflammatory neurological diseases caused by bacteria in ruminants are: listeriosis, suppurative leptomeningitis and meningoencephalitis, cerebral and spinal cord abscesses, basilar empyema and neurotuberculosis.

### EXAMPLES

The present invention will now be described in more detail with reference to the following Examples, which should in no way be construed to be limiting the scope of the present invention.

### Example 1: Effect of flavonoids on perfomance, animal behavior and rumen gene expression in bulls fed high-concentrate diets

### Materials and Methods

### Animals, Feeding, Housing and Experimental Design

This study was conducted in accordance with the Spanish guidelines for experimental animal protection (Royal Decree 53/2013 of February 1^{st} on the protection of animals used for experimentation or other scientific purposes; Boletin Oficial del Estado, 2013). One hundred fourty-four Holstein bulls (164.8 ± 5.91 kg of body weight (BW) and 135 ± 7.2 days of age) were fattened under commercial conditions in a farm (Granja I'Alzina, L'Alzina, Lleida). Animals were randomly allocated in one of eight totally covered pens (12 by 6 m) that were deep-bedded with straw and equipped with a three-space feeder (1.50 m length, 0.40 m width, 1.50 m height, and 0.35 m depth). The feeder of each pen weighed the concentrate continuously as described by Verdú et al. (2017), and these data were recorded to calculate concentrate consumption by pen.

Pens were also equipped with one drinker (0.30 m length, 0.30 m width, 0.18 m depth) and a water meter recording daily pen water consumption. Straw was offered *ad libitum* in a separated straw feeder (3.60 m length, 1.10 m wide, and 0.32 m depth), and every time it was replaced was recorded to estimate the total straw consumption. As straw was also used for bedding, these data are only an estimation.

### Feed Consumption and Performance

Animals were fed a commercial concentrate in meal form, formulated to accomplish the nutritional requirements of the animals (NRC, 2001). The first 112 days of the study, animals were fed a grower concentrate formula, and between 112 days to the end of the study, animals were fed a finisher concentrate. Ingredients and nutritional composition of the concentrates are showed in Table 1. Throughout the study, animals had *ad libitum* access to wheat straw (3.5 % CP, 1.6 % ether extract, 70.9 % NDF, and 6.1 % ash; DM basis) and fresh water.

**Table 1. Ingredients and nutrient composition of the feed concentrates**

| | | Item | Growing | Finishing |
|---|---|---|---|---|
| | | Ingredients, % | | |
| | | Corn grain meal | 39.97 | 45.09 |
| | | Barley grain meal | 17.98 | 15.55 |
| | | DDGs | 17.98 | 15.02 |
| | | Wheat | 10.97 | 11.03 |
| | | Beet pulp | 7.39 | 8.02 |
| | | Palm oil | 2.00 | 2.50 |
| | | Calcium carbonate | 1.55 | 1.28 |
| | | Urea | 0.80 | 0.40 |
| | | Sodium bicarbonate | 0.50 | 0.40 |
| | | Dicalcium phosphate | 0.36 | 0.31 |
| | | Vitamin premix | 0.30 | 0.20 |
| | | Salt | 0.20 | 0.20 |

| | Nutrients, dry matter (DM) basis | | | |
|---|---|---|---|---|
| | | UFc/kg | 1.18 | 1.06 |
| | | Crude protein (CP), % | 15.73 | 12.26 |
| | | Ether extract (EE), % | 5.82 | 5.41 |
| | | Ash, % | 5.60 | 4.38 |
| | | NDF, % | 17.80 | 15.11 |
| | | TDN, % | 88.19 | 79.02 |
| | | PDIE, g/kg | 101.6 | 88.1 |
| | | PDIN, g/kg | 100.7 | 79.9 |
| | | NFC, % | 55.05 | 51.23 |

DM:Dry Matter; UFc: Meat fodder crops unit; CP:Crude Protein; NDF: Neutral Detergent Fibre; TDN: Total Digestive Nutrients; PDIE: Protein Digested Intestine (protein digested in the small intestine when rumenfermentable energy is limiting) - Energy; PDIN: Protein Digested Intestine (protein digested in the small intestine when rumenfermentable nitrogen is limiting) - Nitrogen; NFC: Non-Fiber Carbohydrates.

Animals were randomly allocated to one of 8 pens, and assigned to one of the two treatments (4 pens per treatment), either control (C) or supplemented (BF) with 0.04 % of bitter orange extract *(Citrus aurantium)* of the whole fruit rich in naringin, >20% (Bioflavex^{®} CA, Interquim, S.A., Barcelona, Spain).

Animals were weighed individually every 14 days throughout the study in 12 experimental periods of 14 days, during the 8 first periods (from 1 to 112 days) the animals consumed the growing concentrate and during the last 4 periods (from 113 to 168 days) and during the days before slaughter animals consumed the finishing concentrate (see Table 1). After 168 days of study bulls were transported to the slaughterhouse (Escorxador del Grup Alimentari Guissona, Guissona, Spain), located 15 km from the farm. Animals were slaughtered in two weeks, 4 pens per week, two pens from C and two from BF bulls. The time waiting before slaughter was less than 6 h. Animals were weighed before loading. They were slaughtered by commercial practices and following the EU Regulation 1099/2009 on the protection of animals at the time of killing or slaughtering. Hot carcass weight (HCW) of every animal were recorded.

### Animal Behaviour

A visual scan procedure at days 15, 30, 43, 57, 71, 85, 94, 112, 127, 141, 155, and 170 of the study was performed to study the general activity (standing, lying, eating, drinking, and ruminating) and social behavior (nonagonistic, agonistic, and sexual interactions) of the animals in every pen. Social behavior activities recorded are described in Table 2. The visual observation was made for 2 pens at the same time from 8:00 to 10:30 h am, as described by Mach et al. (2008), Rotger et al. (2006), Robles et al. (2007), and Marti et al. (2010). General activities were scored using 3 scan samplings of 10 s at 5 min intervals, and social behavior was scored during three continuous sampling periods of 5 min. This scanning procedure of 15 min was repeated twice consecutively in each pen, starting randomly in a different pen every scanning day. This method describes a behavior exhibited by an animal at a fixed time interval.

**Table 2. Description of the social behavioural categories recorded.**

| Interactions | Item | | Definition |
|---|---|---|---|
| Nonagonistic interactions | Self-grooming | Nonstereotyped licking of its own body, scratching with a back limb or against the fixtures. | |
| | Social behaviour | Licking, nosing with the muzzle or horning a neighboring bull. | |
| | Oral non-nutritive behaviour | Licking or biting fixtures with non-nutritive finality. | |
| Agonistic interactions | Fighting | When bulls pushed vigorously head against head. | |
| | Butting | When one bull push vigorously its head against any part of another bull's body. | |
| | Displacement | When one bull jostle itself between 2 other bulls or between a bull and any equipment. | |
| | Chasing | When a bull follow fast or run behind another bull. | |
| | Chasing-up | When a bull push a resting animal and make him to stand up. | |
| Sexual interactions | Flehmen | Upper lip reversed. | |
| | Attempted mounts | Head on the back of another animal. | |
| | Completed mounts | Forelimbs on the back of another animal. | |
| Sterertypies | Oral stereotypies | Tongue rolling, stereotyped licking or bitting any equipment | |

### Carcass Quality

After slaughtering, hot carcass weight (HCW) was registered for every animal. Dressing percentage was calculated by dividing HCW by body weight (BW) recorded before slaughtering. And, following the (S)EUROP categories described by the EU Regulation No. 1208/81 and 1026/91, conformation of carcasses was classified, where "E" corresponded to an excellent conformation, "U" to very good conformation, "R" to good conformation, "O" to fair conformation, and "P" to a poor conformation. The fat cover was classified according the EU Regulation No. 1208/81, which utilizes a classification system by numbers, 1.2.3.4.5, where 5 explains a very high degree of covering fat and heavy fat deposits in the thoracic cavity, and 1 is classified as low degree, with no fat cover.

### Rumen and Liver Macroscopic Evaluation and sample collection

Rumen and liver of every animal were macroscopically evaluated at the slaughterhouse. Rumens were classified depending on the color by a visual evaluation, from 1 to 5, being "5" a black colored rumen and "1" a white colored rumen (González et al., 2001). They were also divided into areas according to Lesmeister et al. (2004) to examine the presence of ulcers, baldness regions, and of clumped papillae (Nocek et al., 1984). Liver abscesses were classified according to Brown et al. (1975).

Additionally, 1-cm2 section of each rumen site were sampled and were rinsed 2 times with chilled PBS after sampling and immediately incubated in RNA-later (Invitrogen, Madrid, Spain) to preserve the RNA integrity. After 24 hours of incubation with RNA later at 4 °C, the liquid was removed and tissue was frozen at -80 °C until further RNA extraction and subsequent gene expression analysis.

### Biological and Chemical Analyses

1-cm2 section of each rumen site were sampled and were rinsed 2 times with chilled phosphate-buffered saline (PBS) after sampling and immediately incubated in RNA-later (Invitrogen, Madrid, Spain) to preserve the RNA integrity. After 24 hours of incubation with RNA later at 4 °C, the liquid was removed and tissue was frozen at -80 °C until further RNA extraction and subsequent gene expression analysis.

During the study, samples of concentrate were collected at days 0, 42, 84, 126, and 168 days and analyzed for dry matter (DM) (24 h at 103°C), ash (4 h at 550°C), crude protein (CP) by the Kjeldahl method (method 981.10; AOAC, 1995), acid detergent fibre (ADF) and neutral detergent fibre (NDF) according to Van Soest et al. (1991) using sodium sulfite and alpha-amylase, and ether extract (EE) by Soxhlet with a previous acid hydrolysis (method 920.39; AOAC, 1995).

Naringin was determined for every sample as a Bioflavex^{®} CA marker for BF group, and was used as a quality control analysis to guarantee the correct addition of the product into the feed by Laboratory of Interquim S.A. internal method for naringin quantification using HLPC developed by Interquim S.A. was used (Paniagua et al., 2018).

For gene expression analyses, total RNA was extracted from rumen wall tissues using Trizol (Invitrogen). Isolated mRNA was reverse transcribed to cDNA using an PrimeScript RT Reagent Kit (Takara, Frankfurt, Germany) following the manufacturer's instructions. The RNA purity was assessed by a NanoDrop instrument (ThermoFisher, Madrid, Spain) at 260, 280, and 230 nm. The quantification of expression of genes coding for 1) genes coding the production, expression, and turnover of neurotransmitters: free fatty acid receptor 2 (FFAR2) and free fatty acid receptor 3 (FFAR3), pancreatic polypeptide receptor 1 (*PPYR1*); cholecystokinin B receptor (CCKBR) 2) genes encoding pro-inflammatory cytokines or cytokine IL-25 *(IL25)* and antimicrobial peptides released by intestinal cells, β--defensins, and 3) bitter taste receptors type 2 member 7, 16, 38 and 39 (*TAS2R7, TAS2R16, TAS2R38* and *TAS2R39*) were performed by quantitative PCR (qPCR) using gene codifying for Ribosomal Protein Subunit 9 (*RPS9*) as a housekeeping gene, which was checked for stability following Vandesompele et al. (2002) in comparison with genes codifying for β-actin (*ACTB*)*,* Ubiquitously expressed Transcript protein (UXT) and Glyceraldehyde 3-phosphate dehydrogenase (*GAPDH*)*.* The qPCR conditions for each set of primers were individually optimized. The specificity of the amplification was evaluated by single band identification at the expected molecular weight in 0.8% DNA agarose gels and a single peak in the melting curve. The efficiency was calculated by amplifying serial 1:10 dilutions of each gene amplicon. A standard curve of crossing point (Cq) versus the logarithm of the concentration was plotted to obtain the efficiency, which was calculated using the formula 10^{1/slope}, with an acceptable range of 1.8 to 2.2. A total reaction volume of 20 µL was used, containing 50 ng of cDNA, 10 µL of SYBR Premix EXTaq (TliRNAseH) (Takara, Frankfurt, Germany) and the optimized primer concentration for each gene. The qPCR reactions were cycled as follows: an initial denaturing step of 10 min at 95°C followed by 40 cycles of 10 s at 95°C, 15 s at optimized annealing temperature for each gene, 30 s at 72°C, and a final extension of 10 min at 72°C. The resulting Cq values were used to calculate the relative expression of selected genes by relative quantification using a reference gene (housekeeping gene) and a calibrator of control group.

Ruminal volatile fatty acids (VFA) concentration was analyzed with a semicapillary column (15 m by 0.53 mm i.d., 0.5-µm film thickness, TRB-FFAP; Teknokroma, Barcelona, Spain) composed of 100% polyethylene glycol esterified with nitroterephtalic acid, bonded and crosslinked phase (method number 5560) using a CP-3800-GC (Varian, Inc., Walnut Creek, CA).

### Results

### Animal health

Two animals from the Control group (C group) did not finish the study due to lameness problems, and were removed before day 168. One animal from the BF^{®} CA treated group (BF group or BF bulls) that finished the study was also removed from the database due to chronic health problems. All the data from these animals were removed from database.

### Intake and Water Consumption, Performance and Carcass Quality

No statistical differences were found throughout the study for concentrate intake between treatments (Table 3), neither during the growing phase (Table 4) nor for the finishing phase (Table 5). In the same way, the estimation of the straw consumption did not show statistical differences during all the study (Table 3), neither for the growing phase (Table 4) nor for the finishing phase (Table 5). Water consumption was not affected by treatments throughout the study neither (Table 3), nor during the growing (Table 4) nor for the finishing phase (Table 5).

**Table 3. Performance, concentrate intake, and eating behaviour from 0 d to 168 days of age for Holstein bulls fed high-concentrate diets supplemented with BIOFLAVEX^{®} CA**

| | Treatment¹ | | *P*-value² | | | |
|---|---|---|---|---|---|---|
| Item | Control | BF^{®} CA | SEM⁴ | T | Time | T x Time |
| Initial age, d | 134.25 | 135.22 | 0.215 | 0.002 | | |
| Final age, d | 302.02 | 303.22 | 0.677 | 0.25 | | |
| Initial BW, kg | 165.03 | 164.64 | 5.906 | 0.96 | | |
| Final BW (168 d of study), kg | 436.34 | 439.48 | 1.849 | 0.28 | | |
| CV, % | 8.10 | 8.91 | 0.764 | 0.46 | <.0001 | 0.34 |
| ADG, kg/d | 1.62 | 1.64 | 0.011 | 0.19 | <.0001 | 0.57 |
| CV, % | 27.60 | 26.85 | 1.564 | 0.74 | <.0001 | 0.61 |
| Concentrate DM intake | | | | | | |
| Mean, kg/d | 7.21 | 7.04 | 0.126 | 0.37 | <.0001 | 0.51 |
| CV, % | 10.93 | 11.32 | 0.623 | 0.66 | 0.0002 | 0.97 |
| FCR, kg/kg | 5.36 | 5.11 | 0.108 | 0.10 | <.0001 | 0.03 |

| | | | | | | |
|---|---|---|---|---|---|---|
| ¹ Control = non-supplemented, BF^{®} CA = concentrate supplemented with BIOFLAVEX^{®} CA at 0.04%. ² T = treatment effect; Time = time effect (period of 14 days); T x Time = treatment by time interaction effect. ³ADG = Average daily gain ⁴SEM = standard error of the means of the log-transformed data | | | | | | |

**Table 4. Performance, concentrate intake, and eating behaviour from 4 to 9 months of age for Holstein bulls fed high-concentrate diets supplemented with BIOFLAVEX^{®} CA**

| | Treatment¹ | | *P*-value² | | | |
|---|---|---|---|---|---|---|
| Item | Control | BF^{®} CA | SEM | T | Time | T x Time |
| Initial age, d | 134.25 | 135.22 | 0.215 | 0.002 | | |
| Final age, d | 246.16 | 247.22 | 0.689 | 0.32 | | |
| Initial BW, kg | 165.03 | 164.64 | 5.906 | 0.96 | | |
| Final BW (168 d of study), kg | 360.34 | 360.27 | 1.282 | 0.97 | | |
| CV, % | 8.65 | 9.37 | 0.773 | 0.54 | <.0001 | 0.34 |
| ADG, kg/d | 1.75 | 1.75 | 0.011 | 0.97 | <.0001 | 0.58 |
| CV, % | 19.98 | 22.15 | 0.934 | 0.11 | 0.0011 | 0.29 |
| Concentrate DM intake | | | | | | |
| Mean, kg/d | 6.83 | 6.60 | 0.143 | 0.26 | <.0001 | 0.46 |
| CV, % | 10.53 | 11.29 | 0.700 | 0.45 | 0.0002 | 0.95 |
| FCR, kg/kg | 4.50 | 4.34 | 0.087 | 0.19 | <.0001 | 0.22 |

| | | | | | | |
|---|---|---|---|---|---|---|
| ¹ Control = non-supplemented, BF^{®} CA = concentrate supplemented with BIOFLAVEX^{®} CA at 0.04%. ² T = treatment effect; Time = time effect (period of 14 days); T x Time = treatment by time interaction effect. | | | | | | |

**Table 5. Performance, concentrate intake, and eating behaviour from 9 to 11 months of age for Holstein bulls fed high-concentrate diets supplemented with BIOFLAVEX^{®} CA**

| | Treatment¹ | | *P*-value² | | | |
|---|---|---|---|---|---|---|
| Item | Control | BF^{®} CA | SEM | T | Time | T x Time |
| Initial age, d | 246.16 | 247.22 | 0.689 | 0.32 | | |
| Final age, d | 302.02 | 303.22 | 0.677 | 0.25 | | |
| Initial BW, kg | 360.34 | 360.27 | 1.212 | 0.97 | | |
| Final BW (168 d of study), kg | 436.34 | 439.48 | 1.849 | 0.28 | | |
| CV, % | 7.04 | 8.24 | 0.308 | 0.01 | 0.82 | 0.76 |
| ADG, kg/d | 1.36 | 1.41 | 0.019 | 0.048 | <.0001 | 0.35 |
| CV, % | 42.83 | 36.28 | 1.831 | 0.02 | 0.018 | 0.95 |
| Concentrate DM intake | | | | | | |
| Mean, kg/d | 7.96 | 7.91 | 0.193 | 0.86 | 0.022 | 0.39 |
| CV, % | 11.68 | 11.43 | 0.767 | 0.82 | 0.63 | 0.70 |
| FCR, kg/kg 7.08 | | 6.66 | 0.297 | 0.34 | <.0001 | 0.049 |

| | | | | | | |
|---|---|---|---|---|---|---|
| ¹ Control = non-supplemented, BF^{®} CA = concentrate supplemented with BIOFLAVEX^{®} CA at 0.04%. ² T = treatment effect; Time = time effect (period of 14 days); T x Time = treatment by time interaction effect. | | | | | | |

The average daily gain (ADG) of the growing phase is showed in the Table 4. Non-statistical differences were found for the analyzed productive parameters during this phase. However, ADG during the finishing phase was greater (P < 0.05) for BF bulls (1.41 ± 0.019 kg/d) than for C group (1.36 ± 0.019 kg/d), and CV for this parameter was lesser (P < 0.05) for BF group (36.28 ± 1.831 %) compared with C bulls (42.83 ± 1.831 %) (Table 5). Otherwise, CV for final BW was greater (P = 0.01) for BF bulls (8.24 ± 0.308 %) than for C bulls (7.04 ± 0.308 %). Feed conversion ratio (FCR) tended (P = 0.10) to be lesser for BF bulls (5.11 ± 0.108 kg/kg) than for C bulls (5.36 ± 0.108 kg/kg) at the end of the study, although BW (437.9 ± 1.85 kg) and concentrate intake (7.13 ± 0.126 kg/d) were not affected by treatment (Table 3). An interaction between treatment and time was found for FCR (P = 0.05) during the finishing phase (Table 5).

In summary, BF bulls performed better ADG during this finishing phase without an increase of concentrate intake. Consequently, BF bulls were more efficient during this phase, although FCR improvement was only numerical. Then, assuming that bulls supplemented with flavonoids may have reduced the large meal sizes, this could explain the improvement in concentrate efficiency during the finishing phase and the greater ADG performed of these BF animals.

Carcass quality data are presented in Table 6. Final BW (451.51 ± 3.154 kg), dressing percentage (52.94 % ± 0.326), carcass conformation and fatness score were not affected by treatment.

**Table 6. Carcass quality from Holstein bulls fed high-concentrate diets supplemented with BIOFLAVEX^{®} CA.**

| | | Treatment¹ | | | *P*-value² |
|---|---|---|---|---|---|
| Item | | Control | BF^{®} CA | SEM | T |
| Age before slaughter, d | | 313.38 | 314.75 | 0.916 | 0.29 |
| Days in study, d | | 179.37 | 179.53 | 0.431 | 0.80 |
| BW before slaughter, kg | | 450.39 | 452.62 | 3.154 | 0.62 |
| Hot carcass weight, kg | | 237.60 | 239.92 | 2.019 | 0.42 |
| Dressing percentage, % | | 52.80 | 53.07 | 0.326 | 0.56 |
| Fatness, % | | | | | - |
| | 1 | | | | |
| | 2 | | | | |
| | 3 | 100 | 100 | | |
| Conformation³, % | | | | | 0.37 |
| | P | 91.43 | 84.93 | | |
| | O | 8.57 | 13.70 | | |
| | R | 0 | 1.37 | | |
| | U | | | | |

| | | | | | |
|---|---|---|---|---|---|
| ¹ Control = non-supplemented, BF^{®} CA = concentrate supplemented with BIOFLAVEX^{®} CA at 0.04%. ² T = treatment effect; Time = time effect (period of 14 days); T x Time = treatment by time interaction effect. ³Conformation of carcasses: (S)EUROP categories described by the EU Regulation No. 1208/81 and 1026/91, where "E" corresponds to an excellent conformation, "U" to very good conformation, "R" to good conformation, "O" to fair conformation, and "P" to a poor conformation | | | | | |

### Animal Behaviour

All data for animal behaviour, general activities and active behaviour as well, are showed in Table 7 and Table 8 for growing and finishing phase, respectively.

***General Activities.*** No statistical differences were found in the percentage of animals per pen standing, lying, eating straw and ruminating throughout the visual observation period (2:30 h) for the growing phase (from 0 to 112 days of the study). The percentage of animals eating concentrate was greater (P < 0.01) for BF bulls compared with animals of C group, and the proportion of animals drinking water tended (P < 0.10) to be greater as well for BF bulls than for C bulls during this phase.

For the finishing phase, no differences were found in the proportion of animals per pen standing, lying, eating straw and drinking water during the visual observation period. In this phase, the proportion of animals per pen eating concentrate tended (P < 0.10) to be greater in BF bulls compared with C bulls, and the proportion of animals ruminating in BF group was greater (P < 0.01) than for C bulls.

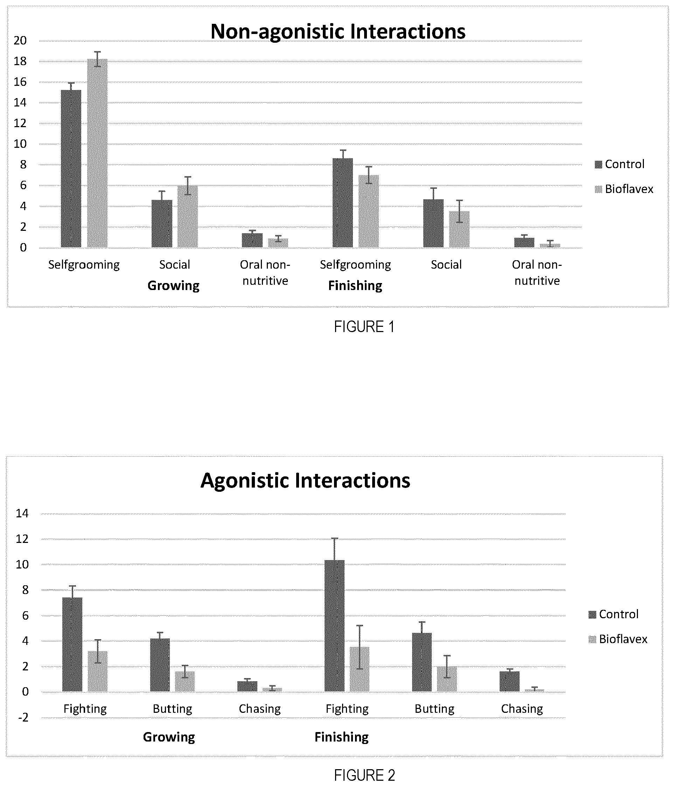

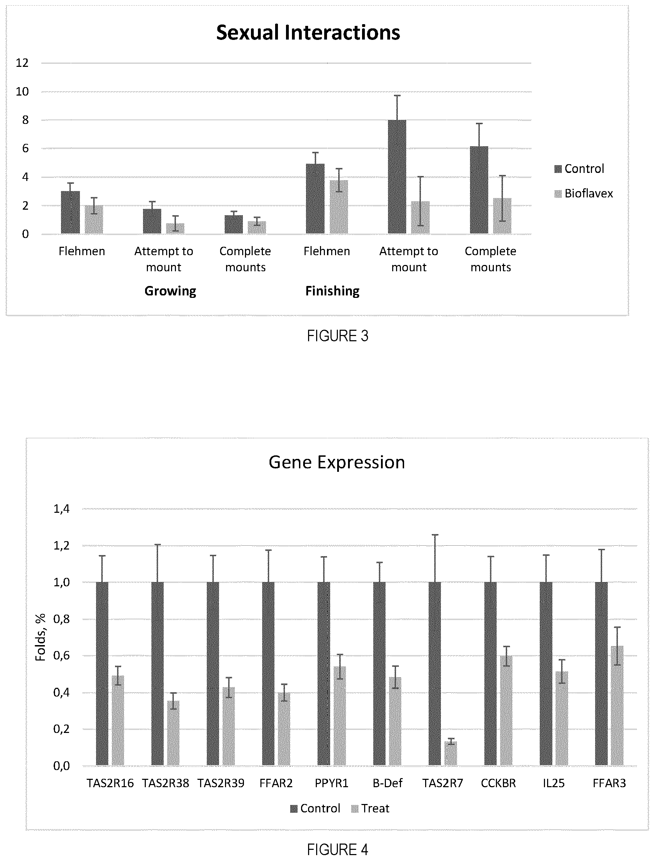

***Active Behaviour.*** In the growing phase, during the visual scan observation period, the only parameter not affected by treatment was the social behaviour. Self-grooming behaviour was greater for BF bulls compared with C group, and this C group exhibited more (P = 0.01) oral non-nutritive behaviour than bulls of the BF group. These results are graphically shown in Figure 1. All behaviour related to agonistic interactions were statistically different during this phase (Figure 2) and more frequently exhibited by bulls of the C group. Fighting behaviour were greater (P < 0.01) in C bulls than in BF bulls, and butting was greater (P < 0.01) for C group compared with BF group as well. Displacement interactions were greater (P < 0.01) exhibited by C group compared with BF group. Chasing and chasing-up interactions were occasionally exhibited, but were greater (P < 0.05) in the C group than in the BF group. Flehmen behaviour was greater (P = 0.05) exhibited in C bulls compared with BF group. Additionally, attempt to mount and complete mounts tended (P < 0.10) to be greater for C bulls than for bulls of the BF group (Figure 3).

During the finishing phase (from 113 to 168 days), no differences between treatments were observed for social and oral behaviour. Bulls from the C group tended (P < 0.10) to perform more self-grooming behaviour than BF bulls (Figure 1). Otherwise, agonistic behaviour, as fighting and butting interactions were greater (P < 0.001 and P < 0.001, respectively) in C group than in BF group. Furthermore, although displacement and chasing rarely occurred, bulls from the C group exhibited greater (P < .0001 in both cases) interactions than BF bulls. Regarding sexual interactions, both attempt to mount and complete mounts were greater (P < 0.001) exhibited for the C group than for BF bulls, whereas flehmen tended (P < 0.10) to be greater for C bulls compared with BF bulls as well. Agonistic and sexual interactions results are graphically shown in Figure 2 and 3.

**Table 7. Behaviour, 1 - 112 days on trial (of the study)**

| Item | | Treatment¹ | | | *P*-values² | | |
|---|---|---|---|---|---|---|---|
| | | Control | BF | SEM³ | T | Time | T x Time |
| General Activity, % | | | | | | | |
| | Standing | 55.64 | 58.80 | 4.123 | 0.60 | <.0001 | 0.99 |
| | Lying | 44.36 | 41.15 | 4.104 | 0.58 | 0.008 | 0.99 |
| | Eating concentrate | 8.95 | 11.07 | 0.495 | <.0001 | <.0001 | 0.82 |
| | Eating straw | 4.87 | 7.45 | 0.617 | 0.35 | 0.188 | 0.44 |
| | Drinking | 1.40 | 2.59 | 0.192 | 0.09 | 0.059 | 0.89 |
| | Ruminating | 12.24 | 14.74 | 2.035 | 0.70 | 0.003 | 0.86 |

| Social behaviour, /15 min | | | | | | | |
|---|---|---|---|---|---|---|---|
| | Selfgrooming | 15.22 | 18.23 | 0.717 | 0.01 | <.0001 | 0.096 |
| | Social | 4.61 | 5.97 | 0.851 | 0.14 | <.0001 | 0.99 |
| | Oral non-nutritive | 1.41 | 0.88 | 0.283 | 0.01 | 0.177 | 0.78 |
| | Fighting | 7.42 | 3.20 | 0.908 | 0.0003 | 0.0004 | 0.91 |
| | Butting | 4.19 | 1.61 | 0.480 | <.0001 | <.0001 | 0.73 |
| | Displacement | 1.69 | 1.09 | 0.109 | 0.0004 | 0.0008 | 0.85 |
| | Chasing | 0.84 | 0.30 | 0.203 | 0.04 | 0.140 | 0.70 |
| | Chasing up | 0.16 | 0.02 | 0.068 | 0.03 | 0.103 | 0.22 |
| | Flehmen | 3.03 | 2.00 | 0.574 | 0.05 | 0.045 | 0.63 |
| | Attempt to mount | 1.76 | 0.75 | 0.524 | 0.09 | <.0001 | 0.31 |
| | Complete mounts | 1.33 | 0.91 | 0.284 | 0.09 | <.0001 | 0.31 |

| | | | | | | | |
|---|---|---|---|---|---|---|---|
| ¹ C = control, BF = concentrate supplemented with BIOFLAVEX^{®} CA at 0.04% ² T = treatment effect; Time = time effect (measurements every 14 days); T x Time = treatment by time interaction. ³ SEM = standard error of the means of the log-transformed data (general activity) or root transformed data (social behaviour). | | | | | | | |

**Table 8. Behaviour, 112 - 168 days on trial (of the study)**

| Item | | Treatment¹ | | | *P*-values² | | |
|---|---|---|---|---|---|---|---|
| | | Control | BF | SEM³ | T | Time | T x Time |
| General Activity, % | | | | | | | |
| | Standing | 63.46 | 64.11 | 2.700 | 0.51 | 0.739 | 0.70 |
| | Lying | 35.44 | 35.68 | 2.673 | 0.75 | 0.881 | 0.86 |
| | Eating concentrate | 6.00 | 7.91 | 0.815 | 0.09 | 0.395 | 0.65 |
| | Eating straw | 4.27 | 6.25 | 0.869 | 0.24 | 0.072 | 0.69 |
| | Drinking | 1.87 | 1.93 | 0.382 | 0.87 | 0.203 | 0.85 |
| | Ruminating | 7.33 | 12.68 | 1.813 | <.0001 | 0.194 | 0.99 |

| Social behaviour, /15 min | | | | | | | |
|---|---|---|---|---|---|---|---|
| | Selfgrooming | 8.62 | 7.00 | 0.809 | 0.08 | 0.480 | 0.80 |
| | Social | 4.69 | 3.53 | 1.067 | 0.24 | 0.451 | 0.92 |
| | Oral non-nutritive | 0.94 | 0.38 | 0.318 | 0.20 | 0.271 | 0.54 |
| | Fighting | 10.34 | 3.53 | 1.712 | <.0001 | 0.0004 | 0.49 |
| | Butting | 4.63 | 2.00 | 0.869 | 0.0004 | 0.046 | 0.50 |
| | Displacement | 0.78 | 0.28 | 0.242 | 0.0009 | 0.143 | 0.91 |
| | Chasing | 1.62 | 0.19 | 0.194 | <.0001 | <.0001 | 0.025 |
| | Chasing up | 0.13 | 0.03 | 0.042 | 0.12 | 0.450 | 0.17 |
| | Flehmen | 4.91 | 3.78 | 0.807 | 0.07 | 0.010 | 0.86 |
| | Attempt to mount | 8.01 | 2.32 | 1.717 | 0.0008 | <.0001 | 0.020 |
| | Complete mounts | 6.16 | 2.52 | 1.610 | 0.0008 | <.0001 | 0.20 |

| | | | | | | | |
|---|---|---|---|---|---|---|---|
| ¹ C = control, BF = concentrate supplemented with BIOFLAVEX^{®} CA at 0.04% ² T = treatment effect; Time = time effect (measurements every 14 days); T x Time = treatment by time interaction. ³ SEM = standard error of the means of the log-transformed data (general activity) or root transformed data (social behaviour). | | | | | | | |

When analyzing eating behaviour visual observation periods (morning) indicated that bulls supplemented with flavonoids had greater occupancy of the concentrate feeder compared with C bulls; this effect was more pronounced during the growing phase than during the finishing phase. Then, during the visual observation period these BF animals devoted more time to eat. In the growing phase, despite this greater occupancy of the feeder, concentrate intake was numerically lesser for BF bulls, indicating that probably eating rate could be lesser. Although non-statistical differences were found for occupancy of the straw feeder (morning visual scan sampling data) throughout the study, BF bulls exhibited greater ruminating behaviour during the finishing phase. It is difficult to explain these result, and it may be due to the visual scan procedure, which does not describe total daily feeder occupancy and ruminating activities. Occupancy of the drinker tended to be greater for bulls supplemented with flavonoids during the growing phase. Usually, dry matter intake and water intake are directly related.

### Rumen fermentation

Rumen fermentation VFA are presented in Table 10. Total VFA concentration in the rumen was not affected by treatment. The molar proportion of acetate was greater (P < .0001) in BF bulls compared with C bulls, whereas molar proportion of propionate was greater (P < .0001) for bulls of the C bulls than for BF bulls. Accordingly, acetate:propionate ratio was greater (P < .0001) for the BF bulls than for C bulls. The remaining of VFA analyzed (butyrate, valerate, isobutyrate and isovalerate) were not affected by the treatment.

**Table 10. Rumen fermentation parameters VFA of Holstein bulls fed high-concentrate diets supplemented or not with Bioflavex CA**

| | | | Control¹ | BF CA² | SEM | *P-value* |
|---|---|---|---|---|---|---|
| Rumen | | | | | | |
| | Total VFA, mM | | 75.6 | 67.2 | 4.76 | 0.22 |
| | Individual VFA, mol/100 mol | | | | | |
| | | Acetate | 58.8 | 66.4 | 1.12 | <.0001 |
| | | Propionate | 28.9 | 20.7 | 1.21 | <.0001 |
| | | Isobutyrate | 7.4 | 7.5 | 0.34 | 0.90 |
| | | n-butyrate | 1.2 | 1.5 | 1.13 | 0.11 |
| | | IsoValerate | 1.5 | 1.3 | 0.13 | 0.26 |
| | | Valerate | 2.1 | 2.5 | 0.24 | 0.22 |
| | | Acetate:propionate, mol/mol | 2.15 | 3.35 | 0.171 | <.0001 |

| | | | | | | |
|---|---|---|---|---|---|---|
| ¹ Control = non-supplemented ² BF CA = concentrate supplemented with BIOFLAVEX^{®} CA at 0.04%. | | | | | | |

### Expression of genes in the rumen

The relative expression ratio of genes studied in the rumen epithelium is presented in Table 11 and Figure 4. The supplementation with flavonoids affected the relative expression ratio of all the bitter taste receptors (TAS2R) analyzed, being lesser expressed by BF bulls compared with C bulls. The relative expression ratio of TAS2R7, TAS2R16, TAS2R38, and TAS2R39 were greater (P = 0.002, P = 0.003, P = 0.002, and P = 0.002, respectively) for bulls of the C group compared with BF bulls.

The relative expression ratio of receptors related with the neurotransmitter signaling differ among receptor type. Whereas the FFAR3 tended (P = 0.10) to be greater for C bulls than for BF bulls, the FFAR2 was greater (P = 0.002) expressed in this C bulls compared with BF bulls. In addition, the relative expression ratio for PPYR1 and CCKBR was greater (P = 0.003 and P = 0.007, respectively) as well for C bulls than for BF bulls.

Furthermore, the relative expression ratio of the receptors related with the inflammation like IL-25, , and β-defensin, were greater (P = 0.005, P = 0.03, and P = 0.0002, respectively) for C bulls than for BF bulls.

**Table 11. Gene expression ratio of Holstein bulls fed high-concentrate diets supplemented or not with Bioflavex CA after 168 days of treatment**

| | Control*¹* | BF CA² | SEM³ | *P-value* |
|---|---|---|---|---|
| *TAS2R7⁴* | 0.94 | 0.13 | 0.139 | 0.002 |
| *TAS2R16⁵* | 0.94 | 0.46 | 0.097 | 0.003 |
| *TAS2R38⁶* | 1.06 | 0.38 | 0.125 | 0.002 |
| *TAS2R39⁷* | 0.88 | 0.37 | 0.100 | 0.002 |
| *FFAR3⁸* | 0.98 | 0.64 | 0.141 | 0.10 |
| *FFAR2⁹* | 0.96 | 0.38 | 0.111 | 0.002 |
| *PPYR1¹⁰* | 1.02 | 0.55 | 0.103 | 0.003 |
| *CCKBR¹¹* | 1.04 | 0.62 | 0.098 | 0.007 |
| *IL-25* | 0.98 | 0.50 | 0.107 | 0.005 |
| β*-Defensin- ß* | 1.00 | 0.48 | 0.085 | 0.0002 |

| | | | | |
|---|---|---|---|---|
| *¹* Control = non-supplemented. *²* BF CA = concentrate supplemented with BIOFLAVEX^{®} CA at 0.04%. *³*SEM are derived from statistical analyses of base 10 log-transformed data, and means are back-transformed values. *⁴ Bitter* taste *receptor 7* *⁵ Bitter* taste *receptor 16* *⁶ Bitter taste receptor 38* *⁷ Bitter taste receptor 39* *⁸* free *fatty acid receptor 3 (gpr41)* *⁹ free fatty acid receptor 2 (gpr43)* *¹⁰ pancreatic polypeptide receptor 1* *¹¹ cholecystokinin receptor 4* | | | | |

### Example 2: Effect of flavonoids supplementation of weanling diets on gene expression of bitter taste receptors (T2Rs) in piglets for the nursery period

### Materials and Methods

### Animals, Feeding, Housing and Experimental Design

A total of 168 commercial crossing piglets ([Large White x Landrace] x Pietrain), were used in the trial. The animals were obtained from the same commercial farm on the day of weaning and moved to the nursery unit (without transport). Male and female 21-d old piglets of 4-7 kg of BW were used. Plastic ear tag identification with the animal's number was used. For the performance trial, animals were distributed into 2 blocks by initial body weight. Within each block pigs were distributed in pens for a balanced body weight distribution. Each block was therefore consisted of 6 pens of 14 animals to which the experimental diets were randomly assigned. Pigs were then allocated in 12 pens (14 piglets/pen/weanling batch). Each pen has had a commercial non-lidded hopper and a nipple waterer to ensure *ad libitum* feeding and free water access. Pens were allotted to two experimental treatments (6 replicates for each treatment).

### Feeding programme

At weaning, the selected animals were offered the same basal pre-starter diets following the same specification but with or without supplementation with the experimental product under study. The pre-starter diet was fed ad libitum for fourteen consecutive days (Table 1, ingredients and Table 2, nutrient composition)

Experimental diet was produced in mash and bagged and labelled, ready for dispatch to the farm. Diets were mixed in batches of 1500kg.

**Table 12. Composition of the pre-starter diet (%)¹**

| INGREDIENTS | % |
|---|---|
| Maize | 18.86 |
| Wheat | 16.25 |
| Acid milk whey | 13.00 |
| Soybean meal HTM96 | 9.38 |
| Barley | 8.81 |
| Extruded Wheat | 7.83 |
| Extruded Maize | 5.22 |
| Soy flour (47% protein) | 4.05 |
| Soybean meal concentrate | 4.00 |
| Fishmeal 70%CP | 4.00 |
| Animal plasma 80CP | 3.00 |
| Lard | 2.90 |
| di-calcium phosphate | 0.96 |
| Lysine sulphate | 0.53 |
| Vit-Min premix | 0.40 |
| DL-Methionine | 0.22 |
| Salt | 0.20 |
| L-Threonin | 0.18 |
| L-Valine | 0.11 |
| L-Tryptophan 0.07 | |

| | |
|---|---|
| ¹Supplied the following per kg of diet: 7,000 IU of vitamin A (acetate); 500 IU of vitamin D3 (cholecalciferol); 250 IU of vitamin D (25-hydroxicholecalciferol); 45 mg of vitamin E; 1 mg of vitamin K3; 1.5 mg of vitamin B1; 3.5 mg of vitamin B2; 1.75 mg of vitamin B6; 0.03 mg of vitamin B12; 8.5 mg of D-pantothenic acid; 22.5 mg of niacin; 0.1 mg of biotin; 0.75 mg of folacin; 20 mg of Fe (chelate of amino acids); 2.5 mg of Cu (sulphate); 7.5 mg of Cu (chelate of glycine); 0.05 mg of Co (sulphate); 40 mg of Zn (oxide); 12.5 mg Zn (chelate of amino acids); 12.5 mg of Mn (oxide); 7.5 of Mn (chelate of glycine); 0.35 mg of I, 0.5 of Se (organic); 0.1 mg of Se (sodium). | |

**Table 13. Nutrient composition of the experimental pre-starter diet (%, as feed basis)**

| NUTRIENTS | % |
|---|---|
| Dry matter | 90.104 |
| Ash | 5.291 |
| Net energy (Kcal/kg) | 2460 |
| Ether Extract | 6.738 |
| Linoleic acid | 1.836 |
| Crude fibre | 2.75 |
| Neutro detergent fibre | 3.914 |
| Starch | 32.465 |
| Sugars | 10.965 |
| Crude Protein | 20.7 |
| Lys | 1.472 |
| Lys dig | 1.368 |
| Met | 0.538 |
| Met dig | 0.51 |
| Cys | 0.372 |
| Cys dig | 0.324 |
| Met+Cys | 0.91 |
| Met+Cys dig | 0.834 |
| Thr | 0.994 |
| Thr dig | 0.899 |
| Trp | 0.328 |
| Trp dig | 0.295 |
| Val | 1.004 |
| Val dig | 0.999 |
| lie | 0.565 |
| lie dig | 0.517 |
| Leu | 1.07 |
| Leu dig | 0.985 |
| Phe | 0.453 |
| Phe dig | 0.408 |
| Tyr | 0.274 |
| Tyr dig | 0.243 |
| Phe+Tyr | 0.727 |
| Phe+Tyr dig | 0.651 |
| His | 0.26 |
| His dig | 0.231 |
| Ca | 63 |
| P Total | 0.65 |
| P dig | 0.36 |
| Na | 0.29 |
| Cl | 0.43 |

### Treatments and experimental design

Two experimental treatments were prepared, control (T1) or supplemented (T2) with 0.03 % of bitter orange extract (*Citrus aurantium*) of the whole fruit rich in naringin, >20% (Bioflavex^{®} CA, Interquim, S.A., Barcelona, Spain). Therefore, the different experimental treatments were as follows:
T1: Basal diet
T2: T1 + bitter orange extract (300g/Tm)

### intestinal tissue sampling

One piglet per pen was euthanized on day 7 post-weaning (suggested to be the most critical period after weaning) and samples of tissue from the jejunum section was collected in RNA later and formaldehyde respectively and kept stored for further analysis.

### Biological analysis

For gene expression analyses, total RNA was extracted from jejunum tissues using Trizol (Invitrogen). Isolated mRNA was reverse transcribed to cDNA using an PrimeScript RT Reagent Kit (Takara, Frankfurt, Germany) following the manufacturer's instructions. The RNA purity was assessed by a NanoDrop instrument (ThermoFisher, Madrid, Spain) at 260, 280, and 230 nm. The quantification of expression of genes coding for bitter taste receptors type 2 member 7,16,38 and 39 (TAS2R7, TAS2R16, TAS2R38 and TAS2R39) were performed by quantitative PCR (qPCR) using gene codifying for Ribosomal Protein Subunit 9 (RPS9) as a housekeeping gene, which was checked for stability following Vandesompele et al. (2002) in comparison with genes codifying for β-actin (ACTB), Ubiquitously expressed Transcript protein (UXT) and Glyceraldehyde 3-phosphate dehydrogenase (GAPDH). The qPCR conditions for each set of primers were individually optimized. The specificity of the amplification was evaluated by single band identification at the expected molecular weight in 0.8% DNA agarose gels and a single peak in the melting curve. The efficiency was calculated by amplifying serial 1:10 dilutions of each gene amplicon. A standard curve of crossing point (Cq) versus the logarithm of the concentration was plotted to obtain the efficiency, which was calculated using the formula 10^{1/slope}, with an acceptable range of 1.8 to 2.2. A total reaction volume of 20 µL was used, containing 50 ng of cDNA, 10 µL of SYBR Premix EXTaq (TliRNAseH) (Takara, Frankfurt, Germany) and the optimized primer concentration for each gene. The qPCR reactions were cycled as follows: an initial denaturing step of 10 min at 95°C followed by 40 cycles of 10 s at 95°C, 15 s at optimized annealing temperature for each gene, 30 s at 72°C, and a final extension of 10 min at 72°C. The resulting Cq values were used to calculate the relative expression of selected genes by relative quantification using a reference gene (housekeeping gene) and a calibrator of control group (Pfaffl, 2004, Eq. [3.5]).

### Results

The relative expression ratio of genes studied in the jejunum epithelium of the piglets is presented in Figure 5. The supplementation with flavonoids affected the relative expression ratio of all the bitter taste receptors (TAS2R) analyzed, being greater expressed by piglets of the T2 compared with the animals of the T1.

### Example 3: Effect of flavonoids on duodenum epithelium gene expression in bulls fed in high-fat finishing diets

### Materials and Methods

### The same as in the Example 1

### Feed Consumption and Performance

Animals were fed a commercial concentrate in meal form, formulated to accomplish the nutritional requirements of the animals (FEDNA, 2008). The first 112 days of the study, animals were fed a grower concentrate formula, and between 112 days to the end of the study, animals were fed a finisher high-fat concentrate. Ingredients and nutritional composition of the concentrates are showed in Table 14. Throughout the study, animals had *ad libitum* access to wheat straw (3.5 % CP, 1.6 % ether extract, 70.9 % NDF, and 6.1 % ash; DM basis) and fresh water.

**Table 14. Ingredients and nutrient composition of the feed concentrates**

| | Item | | Growing¹ | Finishing² |
|---|---|---|---|---|
| | Ingredient, g/ kg | | | |
| | | Corn grain meal | 399.7 | 436.9 |
| | | Barley grain meal | 179.8 | 150.2 |
| | | DDGs | 179.8 | 150.2 |
| | | Wheat | 109.7 | 109.8 |
| | | Beet pulp | 73.9 | 80.0 |
| | | Palm oil | 20.0 | 45.0 |
| | | Calcium carbonate | 15.5 | 12.8 |
| | | Urea | 8.0 | 4.0 |
| | | Sodium bicarbonate | 5.0 | 4.0 |
| | | Dicalcium phosphate | 3.6 | 3.1 |
| | | Vitamin premix | 3.0 | 2.0 |
| | | Salt | 2.0 | 2.0 |
| Nutrient | | | | |
| | | UFc/kg DM | 1.18 | 1.25 |
| | | CP, g/ kg DM | 157 | 136 |
| | | Ether extract, g/ kg DM | 58 | 84 |
| | | Ash, g/ kg DM | 56 | 46 |
| | | NFD, g/ kg DM | 178 | 169 |
| | | NFC, g/ kg DM | 551 | 565 |

| | | | | |
|---|---|---|---|---|
| ¹ from 0 to 112 days of the study. ² from 113 days to the end of the study. | | | | |

DM:Dry Matter; ME metabolic energy; CP:Crude Protein; NDF: Neutral Detergent Fibre; NFC: Non-Fiber Carbohydrates.

Animals were randomly allocated to one of 8 pens, and assigned to one of the two treatments (4 pens per treatment), either control (C) or supplemented (BF) with 0.04 % of bitter orange extract *(Citrus aurantium)* of the whole fruit rich in naringin, >20% (Bioflavex^{®} CA, Interquim, S.A., Barcelona, Spain).

Animals were weighed individually every 14 days throughout the study in 12 experimental periods of 14 days, during the 8 first periods (from 1 to 112 days) the animals consumed the growing concentrate and during the last 4 periods (from 113 to 168 days) and during the days before slaughter animals consumed the finishing concentrate (see Table 14). After 168 days of study bulls were transported to the slaughterhouse (Escorxador del Grup Alimentari Guissona, Guissona, Spain), located 15 km from the farm. Animals were slaughtered in two weeks, 4 pens per week, two pens from C and two from BF bulls. The time waiting before slaughter was less than 6 h. Animals were weighed before loading. They were slaughtered by commercial practices and following the EU Regulation 1099/2009 on the protection of animals at the time of killing or slaughtering. Hot carcass weight (HCW) of every animal were recorded.

### Biological and Chemical Analyses

1-cm2 section of each dudodenum site were sampled and were rinsed 2 times with chilled phosphate-buffered saline (PBS) after sampling and immediately incubated in RNA-later (Invitrogen, Madrid, Spain) to preserve the RNA integrity. After 24 hours of incubation with RNA later at 4 °C, the liquid was removed and tissue was frozen at -80 °C until further RNA extraction and subsequent gene expression analysis.

For gene expression analyses, total RNA was extracted from rumen wall tissues using Trizol (Invitrogen). Isolated mRNA was reverse transcribed to cDNA using an PrimeScript RT Reagent Kit (Takara, Frankfurt, Germany) following the manufacturer's instructions. The RNA purity was assessed by a NanoDrop instrument (ThermoFisher, Madrid, Spain) at 260, 280, and 230 nm. The quantification of expression of genes coding for 1) genes coding the production, expression, and turnover of neurotransmitters: free fatty acid receptor 2 (FFAR2) and free fatty acid receptor 3 (FFAR3), pancreatic polypeptide receptor 1 (*PPYR1*); cholecystokinin B receptor (CCKBR) 2) genes encoding pro-inflammatory cytokines or cytokine IL-25 (*IL25*) and antimicrobial peptides released by intestinal cells, β--defensins, and 3) bitter taste receptors type 2 member 7, 16, 38 and 39 (*TAS2R7, TAS2R16, TAS2R38* and *TAS2R39*) were performed by quantitative PCR (qPCR) using gene codifying for Ribosomal Protein Subunit 9 (*RPS9*) as a housekeeping gene, which was checked for stability following Vandesompele et al. (2002) in comparison with genes codifying for β-actin (ACTB), Ubiquitously expressed Transcript protein (UXT) and Glyceraldehyde 3-phosphate dehydrogenase (*GAPDH*)*.* The qPCR conditions for each set of primers were individually optimized. The specificity of the amplification was evaluated by single band identification at the expected molecular weight in 0.8% DNA agarose gels and a single peak in the melting curve. The efficiency was calculated by amplifying serial 1:10 dilutions of each gene amplicon. A standard curve of crossing point (Cq) versus the logarithm of the concentration was plotted to obtain the efficiency, which was calculated using the formula 10^{1/slope}, with an acceptable range of 1.8 to 2.2. A total reaction volume of 20 µL was used, containing 50 ng of cDNA, 10 µL of SYBR Premix EXTaq (TliRNAseH) (Takara, Frankfurt, Germany) and the optimized primer concentration for each gene. The qPCR reactions were cycled as follows: an initial denaturing step of 10 min at 95°C followed by 40 cycles of 10 s at 95°C, 15 s at optimized annealing temperature for each gene, 30 s at 72°C, and a final extension of 10 min at 72°C. The resulting Cq values were used to calculate the relative expression of selected genes by relative quantification using a reference gene (housekeeping gene) and a calibrator of control group.

### Results

Regarding the relative expression at mRNA level of genes studied in the duodenum epithelium, data are presented in Figure 6. The supplementation with flavonoids affected the expression of all the bitter taste receptors (TAS2R) analyzed except TAS2R38. In duodenum the relative expression of TAS2R7, TAS2R16, and TAS2R39 were greater (P < 0.001) in C bulls compared with BF bulls. The relative expression of some receptors related with the neurotransmitter signaling also differ among treatments. The FFAR2 (P < 0.01) were greater expressed in this C bulls compared with BF bulls. Furthermore, the relative expression for PPYR1 and CCKBR was greater (P < 0.01) as well for C bulls than for BF bulls. Additionally, the relative expression of the receptors related with the inflammation IL-25, and β-defensin, were again greater (P < 0.05) for C bulls than for BF bulls.

The gene expression of these proinflammatory molecules in the duodenum epithelium of BF bulls was reduced, as observed in the rumen epithelium in the previous study (Example 1). These results were also in agreement with the reduction of the nutrient sensing receptors studied in the duodenum epithelium of BF bulls, such as TAS2R, FFAR2, PPYR1, and CCKBR.

In summary, flavonoid supplementation differently modified gene expression of genes in the duodenum epithelium that could be related with eating pattern and animal behavior regulation.

### REFERENCES

Brown, H., Bing, R.F., Grueter, H.P., McAskill, J.W., Cooley, C.O., Rathmacher, R.P., 1975. Tylosin and chloretetracycline for the prevention of liver abscesses, improved weight gains and feed efficiency in feedlot cattle. J. Anim. Sci. 40, 207-213.
González, J.M., Garcia de Jalon, J.A., Askar, A.R., Guada, J.A., Ferrer, L.M., de las Heras, M., 2001. Efecto de la dieta con cebada y nucleo proteico sobre la patologia ruminal en corderos. In: XXVI Jornadas de la SEOC. Sevilla, Spain. pp. 733-739.
Lesmeister, K.E., Tozer, P.R., Heinrichs, a J., 2004. Development and analysis of a rumen tissue sampling procedure. J. Dairy Sci. 87, 1336-1344. https://doi.org/10.3168/jds.S0022-0302(04)73283-X.
Nocek, J.E., Heald, C.W., Polan, C.E., 1984. Influence of ration physical form and nitrogen availability on ruminal morphology of growing bull calves. J. Dairy Sci. 67, 334-343. https://doi.org/10.3168/jds.S0022-0302(84)81306-5.
Mach, N., Bach, A., Realini, C.E., Font i Furnols, M., Velarde, A., Devant, M., 2009. Burdizzo pre-pubertal castration effects on performance, behaviour, carcass characteristics, and meat quality of Holstein bulls fed high-concentrate diets. Meat Sci. 81, 329-334. https://doi.org/10.1016/j.meatsci.2008.08.007.
Marti, S., Velarde, A., de la Torre, J.L., Bach, A., Aris, A., Serrano, A., Manteca, X., Devant, M., 2010. Effects of ring castration with local anesthesia and analgesia in Holstein calves at 3 months of age on welfare indicators. J. Anim. Sci. 88, 2789-2796. https://doi.org/10.2527/jas.2009-2408.
Paniagua, M., Crespo, J., Bach, A., Devant, M., 2018. Effects of flavonoids extracted from Citrus aurantium on performance, eating and animal behavior, rumen health, and carcass quality in Holstein bulls fed high-concentrate diets. Anim. Feed Sci. Technol. 246, 114-126
Robles, V., González, L.A., Ferret, A., Manteca, X., Calsamiglia, S., 2007. Effects of feeding frequency on intake, ruminal fermentation, and feeding behavior in heifers fed high-concentrate diets. J. Anim. Sci. 85, 2538-2547.
Rotger, A., Ferret, A., Manteca, X., Ruiz De La Torre, J.L., Calsamiglia, S., 2006. Effects of dietary nonstructural carbohydrates and protein sources on feeding behavior of tethered heifers fed high-concentrate diets. J. Anim. Sci. 84, 1197-1204.
Vandesompele J, De Prefer K, Pattyn F, Poppe B, Van Roy N, De Paepe A, Speleman F. Accurate normalization of real-time quantitative RT-PCR data by geometric averaging of multiple internal control genes, Genome Biology, 2002, vol. 3 RESEARCH0034
Van Soest, P.J., Robertson, J.B., Lewis, B.A., 1991. Methods for dietary fiber, neutral detergent fiber, and nonstarch polysaccharides in relation to animal nutrition. J. Dairy Sci. 74, 3583-3597. https://doi.org/10.3168/jds.S0022-0302(91)78551-2.
Verdú, M., Bach, A., Devan, M., 2017. Effect of feeder design and concentrate presentation form on performance, carcass characteristics, and behavior of fattening Holstein bulls fed high-concentrate diets. Anim. Feed Sci. Technol.

## Claims

1. Non-therapeutic use of naringin, naringenin or mixtures thereof for the targeted modulation of the gene expression of at least one bitter taste receptor and/or at least one gut-brain axis receptor to modify the eating pattern and rumination in ruminants or for the targeted modulation of the gene expression of at least one bitter taste receptor to modify the eating patern in pigs, wherein the non-therapeutic use comprises administering naringin, naringenin or mixtures thereof, wherein the amount of naringin, naringenin or mixtures thereof is administered as a mixture with feed, wherein said feed composition comprises at least 0.005% w/w of naringin or the equimolar amount of naringenin or of a mixture of naringin and naringenin if both are present, whereby methods for treatment of the human or animal body by surgery or therapy are excluded.

2. The non-therapeutic use according to claim 1, wherein the bitter taste receptor is selected from the group consisting of TAS2R7, TAS2R16, TAS2R38 and TAS2R39

3. The non-therapeutic use according to claim 1, wherein the gut-brain axis receptor is selected from the group consisting of FFAR2, FFAR3, PPYR1 and CCKBR.

4. The non-therapeutic use according to any one of the preceding claims, wherein the gene expression of at least one said receptor is reduced.

5. The non-therapeutic use according to any one of the preceding claims, wherein naringin or naringenin or mixtures thereof are administered in a daily dose of 0.001 to 0.01 g of naringin per kg body weight of the animal or the equimolar amount of naringenin or of a mixture of naringin and naringenin if both are present.

6. The non-therapeutic use according to any one of the preceding claims, wherein naringin, naringenin or mixtures thereof is in the form of a natural plant extract, preferably said natural plant extract is bitter orange extract.

7. The non-therapeutic use according to the preceding claim, wherein said natural plant extract is bitter orange extract which is administered in a daily dose of 0.005 g per kg body weight of the animal to 0.02 g per kg body weight of the animal.

8. The non-therapeutic use according to any one of the preceding claims comprising administering naringin, naringenin or mixtures thereof to a ruminant and/or pigs for at least 5 days.

9. Naringin, naringenin or mixtures thereof for use in the modulation of the gene expression of at least one immune-related gene in ruminants and/or pigs, wherein said product is administered as a mixture with feed, wherein said feed composition comprises at least 0.005% w/w of naringin or the equimolar amount of naringenin or of a mixture of naringin and naringenin if both are present.

10. The product for use according to the preceding claim, wherein the immune-related gene is selected from the group consisting of IL-6, IL-8, IL-10, IL-25 and β-defensin.

11. The product for use according to any one of the claims 9 to 10, wherein the gene expression of at least one said receptor is reduced.

12. The product for use according to any one of the claims 9 to 11, wherein the product is administered in a daily dose of 0.001 to 0.01 g of naringin per kg body weight of the animal or the equimolar amount of naringenin or of a mixture of naringin and naringenin if both are present.

13. The product for use according to any one of the claims 9 to 12 comprising administering said product to ruminants and/or pigs for at least 5 days.

## Patentansprüche

1. Nicht-therapeutische Verwendung von Naringin, Naringenin oder Mischungen davon zur gezielten Modulation der Genexpression von mindestens einem Bittergeschmacksrezeptor und/oder mindestens einem Darm-Hirn-Achsenrezeptor zur Veränderung des Essverhaltens und der Wiederkäuung bei Wiederkäuern oder zur gezielten Modulation der Genexpression von mindestens einem Bittergeschmacksrezeptor zur Veränderung des Essverhaltens bei Schweinen, wobei die nicht-therapeutische Verwendung die Verabreichung von Naringin, Naringenin oder Mischungen davon umfasst, wobei die Menge an Naringin, Naringenin oder Mischungen davon als Mischung mit Futter verabreicht wird, wobei die Futterzusammensetzung mindestens 0,005 Gew.-/Gew.- % Naringin oder die äquimolare Menge an Naringenin oder einer Mischung aus Naringin und Naringenin umfasst, wenn beide vorhanden sind, wobei Verfahren zur chirurgischen oder therapeutischen Behandlung des menschlichen oder tierischen Körpers ausgeschlossen sind.

2. Die nicht-therapeutische Verwendung nach Anspruch 1, wobei der bittere Geschmacksrezeptor ausgewählt ist aus der Gruppe bestehend aus TAS2R7, TAS2R16, TAS2R38 und TAS2R39.

3. Die nicht-therapeutische Verwendung nach Anspruch 1, wobei der Darm-Hirn-Achsenrezeptor ausgewählt ist aus der Gruppe bestehend aus FFAR2, FFAR3, PPYR1 und CCKBR.

4. Die nicht-therapeutische Verwendung nach einem der vorhergehenden Ansprüche, wobei die Genexpression von mindestens einem der Rezeptoren reduziert ist.

5. Die nicht-therapeutische Verwendung nach einem der vorhergehenden Ansprüche, wobei das Naringin oder das Naringenin oder Mischungen davon in einer täglichen Dosis von 0,001 bis 0,01 g Naringin pro kg Körpergewicht des Tieres oder der äquimolaren Menge von Naringenin oder einer Mischung von Naringin und Naringenin, wenn beide vorhanden sind, verabreicht werden.

6. Die nicht-therapeutische Verwendung nach einem der vorhergehenden Ansprüche, wobei das Naringin, das Naringenin oder Mischungen davon in der Form eines natürlichen Pflanzenextrakts vorliegt, wobei der natürliche Pflanzenextrakt vorzugsweise Bitterorangenextrakt ist.

7. Die nicht-therapeutische Verwendung nach dem vorhergehenden Anspruch, wobei der natürliche Pflanzenextrakt ein Bitterorangenextrakt ist, welcher in einer Tagesdosis von 0,005 g pro kg Körpergewicht des Tieres bis 0,02 g pro kg Körpergewicht des Tieres verabreicht wird.

8. Die nicht-therapeutische Verwendung nach einem der vorhergehenden Ansprüche, umfassend die Verabreichung von Naringin, Naringenin oder Mischungen davon an einen Wiederkäuer und/oder Schweine für mindestens 5 Tage.

9. Naringin, Naringenin oder Mischungen davon zur Verwendung bei der Modulation der Genexpression von mindestens einem immunverwandten Gen bei Wiederkäuern und/oder Schweinen, wobei das Produkt als Mischung mit Futter verabreicht wird, wobei die Futterzusammensetzung mindestens 0,005 Gew.-% Naringin oder die äquimolare Menge an Naringenin oder einer Mischung aus Naringin und Naringenin umfasst, wenn beide vorhanden sind.

10. Das Produkt zur Verwendung nach dem vorhergehenden Anspruch, wobei das immunverwandte Gen ausgewählt ist aus der Gruppe bestehend aus IL-6, IL-8, IL-10, IL-25 und β-Defensin.

11. Das Produkt zur Verwendung nach einem der Ansprüche 9 bis 10, wobei die Genexpression von mindestens einem Rezeptor reduziert ist.

12. Das Produkt zur Verwendung nach einem der Ansprüche 9 bis 11, wobei das Produkt in einer täglichen Dosis von 0,001 bis 0,01 g Naringin pro kg Körpergewicht des Tieres oder der äquimolaren Menge an Naringenin oder einer Mischung aus Naringin und Naringenin, wenn beide vorhanden sind, verabreicht wird.

13. Das Produkt zur Verwendung nach einem der Ansprüche 9 bis 12, umfassend die Verabreichung des Produkts an Wiederkäuer und/oder Schweine für mindestens 5 Tage.

## Revendications

1. Utilisation non thérapeutique de la naringine, de la naringénine ou des mélanges de celles-ci pour la modulation ciblée de l'expression génique d'au moins un récepteur du goût amer et/ou d'au moins un récepteur de l'axe intestin-cerveau pour modifier le schéma alimentaire et la rumination chez les ruminants ou pour la modulation ciblée de l'expression génique d'au moins un récepteur du goût amer pour modifier le schéma alimentaire chez les porcs, dans laquelle l'utilisation non thérapeutique comprend l'administration de naringine, de naringénine ou de leurs mélanges, dans laquelle la quantité de naringine, de naringénine ou des mélanges de celles-ci est administrée en tant que mélange avec des aliments, dans laquelle ladite composition d'aliments comprend au moins 0,005 % p/p de naringine ou la quantité équimolaire de naringénine ou d'un mélange de naringine et de naringénine, si toutes les deux sont présentes, dans laquelle les méthodes de traitement du corps humain ou animal par chirurgie ou thérapie sont exclues.

2. L'utilisation non thérapeutique selon la revendication 1, dans laquelle le récepteur du goût amer est choisi dans le groupe constitué par TAS2R7, TAS2R16, TAS2R38 et TAS2R39.

3. L'utilisation non thérapeutique selon la revendication 1, dans laquelle le récepteur de l'axe intestin-cerveau est choisi dans le groupe constitué par FFAR2, FFAR3, PPYR1 et CCKBR.

4. L'utilisation non thérapeutique selon l'une quelconque des revendications précédentes, dans laquelle l'expression génique d'au moins un dudit récepteur est réduite.

5. L'utilisation non thérapeutique selon l'une quelconque des revendications précédentes, dans laquelle la naringine ou la naringénine ou des mélanges de celles-ci sont administrées à une dose quotidienne de 0,001 à 0,01 g de naringine par kg de poids corporel de l'animal ou la quantité équimolaire de naringénine ou d'un mélange de naringine et de naringénine si toutes les deux sont présentes.

6. L'utilisation non thérapeutique selon l'une quelconque des revendications précédentes, dans laquelle la naringine, la naringénine ou des mélanges de celles-ci est sous la forme d'un extrait naturel de plante, de préférence ledit extrait naturel de plante est un extrait d'orange amère.

7. L'utilisation non thérapeutique selon la revendication précédente, dans laquelle ledit extrait végétal naturel est un extrait d'orange amère qui est administré à une dose quotidienne de 0,005 g par kg de poids corporel de l'animal à 0,02 g par kg de poids corporel de l'animal.

8. L'utilisation non thérapeutique selon l'une quelconque des revendications précédentes comprenant l'administration de naringine, de naringénine ou de mélanges de ceux-ci à un ruminant et/ou à des porcs pendant au moins 5 jours.

9. La naringine, la naringénine ou des mélanges de celles-ci pour l'utilisation dans la modulation de l'expression génique d'au moins un gène lié à l'immunité chez les ruminants et/ou les porcs, dans lequel ledit produit est administré comme un mélange avec des aliments, dans lequel ladite composition d'aliments comprend au moins 0,005 % p/p de naringine ou la quantité équimolaire de naringénine ou d'un mélange de naringine et de naringénine si toutes les deux sont présentes.

10. Le produit pour l'utilisation selon la revendication précédente, dans lequel le gène lié à l'immunité est choisi dans le groupe constitué par l'IL-6, l'IL-8, l'IL-10, l'IL-25 et la β-défensine.

11. Le produit pour l'utilisation selon l'une quelconque des revendications 9 à 10, dans lequel l'expression génique d'au moins un desdits récepteurs est réduite.

12. Le produit pour l'utilisation selon l'une quelconque des revendications 9 à 11, dans lequel le produit est administré à une dose quotidienne de 0,001 à 0,01 g de naringine par kg de poids corporel de l'animal ou la quantité équimolaire de naringénine ou d'un mélange de naringine et de naringénine si toutes les deux sont présentes.

13. Le produit pour l'utilisation selon l'une quelconque des revendications 9 à 12 comprenant l'administration dudit produit à des ruminants et/ou des porcs pendant au moins 5 jours.
